# FASCICULE DE BREVET EUROPEEN

(11) **EP 0 699 472 B1**
(45) Date de publication et mention de la délivrance du brevet: **19.08.1998**
(21) Numéro de dépôt: 95401396.7
(22) Date de dépôt: 15.06.1995
(51) Int. Cl.: B01F 17/56, C11D 1/66, C13K 13/00, C07H 15/04, A61K 7/50

(54) **Procédé de préparation d'agents tensioactifs à partir de sous-produits du blé et leurs applications**
Verfahren zur Herstellung von Tensiden aus Weizennebenprodukten und ihre Verwendungen
Process for the production of surfactants from wheat by-products and their uses

(30) Priorité: 30.08.1994 FR 9410406
(43) Date de publication de la demande: 06.03.1996
(73) Titulaire: AGRO INDUSTRIE RECHERCHES ET DEVELOPPEMENTS (A.R.D.), F-51100 Pomacle (FR)
(72) Inventeur: Bertho, Jean-Noel, F-51100 Reims (FR); Mathaly, Philippe, F-51100 Reims (FR); Dubois, Véronique, F-51100 Reims (FR); De Baynast de Septfontaines, Régis, F-78000 Versailles (FR)
(74) Mandataire: Eidelsberg, Victor Albert

(56) Documents cités:
- EP-A- 0 265 111
- EP-A- 0 507 047
- WO-A-94/21655
- FR-A- 2 328 795
- FR-A- 2 679 563
- US-A- 3 839 318
- US-A- 4 350 766
- US-A- 4 950 743

## Description

La présente invention se rapporte aux procédés de préparation de mélanges de pentosides d'alkyle, à partir de sous-produits du blé, à leurs applications et à leurs formulations notamment en tant qu'agents tensioactifs.

Le greffage de groupes alkyles sur des glucides conduit à des agents tensioactifs dont les propriétés de surface sont très intéressantes et dont la biodégradabilité est généralement bonne (R.D. Swisher, "Surfactant biodégradation", Marcel Dekker, Inc. NEW YORK, 1987).

La réaction la plus couramment rencontrée constitue la glycosylation d'alcools gras par des pentoses ou des glycosides intermédiaires. Celle-ci est réalisée en présence d'un catalyseur acide et les glycosides d'alkyle obtenus sont stables dans un large domaine de pH, notamment neutre et basique. Le substrat le plus souvent utilisé comme sucre réducteur est le glucose. La glycosylation réalisée à partir de ce sucre conduit à des mélanges de glucosides et de polyglucosides d'alkyle qui sont connus pour leurs propriétés tensioactives. Ceux-ci présentent désormais de nombreuses applications dans des domaines aussi variés que la détergence, les industries chimiques et parachimiques, ou encore dans le domaine médical et la biologie (voir par exemple WO 93 07160, WO 93 07249, DE 42 12 080 A1, US 4 987 225).
Pour la préparation de ces glucosides d'alkyle, il est nécessaire de disposer de grandes quantités de glucose. L'obtention de ce glucose à partir, par exemple, de la farine de blé nécessite de nombreuses étapes. Il est en effet indispensable d'éliminer le gluten et de purifier le glucose notamment par des étapes de filtration et de chromatographie sur résines échangeuses d'ions.

L'inconvénient majeur de cette approche réside dans le coût élevé de la matière première lié notamment à l'utilisation de matière agricole noble telle que la farine et aux nombreuses étapes de purification du glucose. Tout ceci limite naturellement les applications potentielles de ces procédés. En outre, la réaction de glycosylation du glucose ou autres hexoses s'effectue à haute température (100 à 150°C), ce qui donne une coloration des produits.

Les documents EP-A-0 507 047 et US-A-3 839 318 décrivent la synthèse de pentosides par réaction d'un pentose avec des alcools gras.

L'invention vise à obtenir des agents tensioactifs à partir d'une matière première particulièrement bon marché par des stades opératoires, simples et peu nombreux, qui ne nécessitent pas l'application de températures élevées induisant une coloration.

Suivant l'invention, on utilise du son ou de la fibre de blé ou encore de la paille de blé comme matière première pour la préparation d'agents tensioactifs.

On peut utiliser du son de blé tel quel, mais de préférence on utilise du son de blé désamylacé, tel que décrit à la demande de brevet européen N°401 117.

On peut également utiliser de la fibre de blé. On entend par fibre de blé les composés obtenus au cours d'un procédé de fractionnement, visant notamment à produire de l'amidon et du gluten selon le protocole suivant :
Le blé broyé au moulin conduit à de la farine et des sons. Le taux d'extraction relativement élevé (de 75 à 90 %) provoque le déplacement d'une partie des sons dans la farine. Une pâte est ensuite préparée par mélange de la farine avec de l'eau, à température ambiante, et à une teneur en matière sèche voisine de 45 %. La pâte est ensuite rediluée aux alentours de 35 % de matière sèche, puis on effectue la séparation des trois composés essentiels que sont l'amidon, le gluten, et les solubles. Cette étape de séparation peut être réalisée par une décantation trois phases, ou par tout autre procédé tel que les procédés MARTIN et dérivés. L'amidon est ensuite tamisé pour le séparer des particules de sons qui s'y trouvent mélangées. Le refus du tamis est donc essentiellement composé de sons et d'amidon résiduel. Dans la présente invention de tels mélanges sont appelés fibres de blé. On appelle également fibres de blé les fractions provenant du tamisage du gluten des effluents, comprenant également des sons et de l'amidon. Plus généralement, selon la présente invention, on appelle fibre de blé, toute matière provenant de la transformation du blé et composée au moins de sons et éventuellement d'amidon. Le son est lui-même composé d'hémicellulose constitué de monomères xylose et arabinose, et de cellulose.

L'invention vise également un procédé de préparation d'agents tensioactifs caractérisé en ce qu'il consiste à mettre du son ou de la fibre de blé, ou encore de la paille de blé en contact avec une solution aqueuse acide entre 20 et 150°C, et de préférence entre 60 et 120°C, pendant au moins cinq secondes pour obtenir un sirop de pentoses, si cela est nécessaire à débarrasser le sirop de pentoses d'un marc solide et à mettre le sirop de pentoses exempt de marc en contact avec un alcool ayant de 6 à 22 atomes de carbone à une température comprise entre 20 et 150°C, et de préférence entre 30 et 110°C, jusqu'à obtention d'une solution de pentosides tensioactifs, et à séparer les pentosides tensioactifs de cette solution.

Ce procédé présente l'avantage d'utiliser des sucres actuellement mal valorisés, de travailler à partir de mélanges de ces sucres et par conséquent d'éviter des étapes de purification.

Par sirop de pentoses, on entend un sirop qui dans le cas de la paille et du son désamylacé contient d'une part, 60 à 75 % de pentoses et d'autre part, 40 à 25% d'hexoses. On appelle aussi sirop de pentoses, un sirop qui peut parfois contenir jusqu'à 65 % de glucose lorsqu'on traite de la fibre et du son de blé non désamylacé, mais qui contient néanmoins une forte proportion de pentoses.

Le sirop est constitué majoritairement de pentoses, notamment de D-xylose et de L-arabinose qui présentent l'avantage d'être plus réactifs lors de la glycosylation. Cette propriété permet un greffage dans des conditions ménagées évitant notamment toute dégradation des réactifs ou des produits de la réaction. Contrairement à la préparation des glucosides et polyglucosides d'alkyle qui nécessite des températures généralement supérieures à 120 °C (voir DE 42 12 080, WO 93 07160), la synthèse des pentosides d'alkyle selon la présente invention peut être réalisée à une température inférieure à 100°C.

De plus, la réaction de glycosylation peut être réalisée sans apport de catalyseurs acides supplémentaires ; l'acidité du sirop permet en effet la catalyse de la réaction.

Le premier stade du procédé suivant l'invention consiste à mettre du son ou de la fibre de blé, ou encore de la paille de blé en contact avec une solution aqueuse acide. On extrait, solubilise et hydrolyse ainsi en milieu aqueux en présence d'acide les polysaccharides présents dans la fibre ou le son de blé brut, désamidonné ou délignifié, dans la paille. L'acide utilisé pouvant être l'acide chlorhydrique, l'acide sulfurique, l'acide phosphorique, un acide sulfonique tel que l'acide benzènesulfonique, l'acide paratoluènesulfonique, l'acide camphresulfonique, l'acide sulfosuccinique ou un sulfosuccinate d'alkyle tel que le sulfosuccinate de décyle ou de lauryle, les acides perhalohydriques, tels que par exemple l'acide perchlorique.

On effectue généralement cette mise en contact pendant 5 à 90 minutes dans un mélangeur simple. Mais on peut ramener la durée de contact à quelques secondes en opérant dans un dispositif permettant d'effectuer des mélanges en ligne, connu sous le nom de "jet cooker".

Lorsque l'on traite du son ou de la paille de blé, le deuxième stade du procédé suivant l'invention consiste à débarrasser le sirop de pentoses d'un marc solide. On effectue avantageusement cette opération par pressage sous une pression de l'ordre de 2.10⁵ à 500.10⁵ Pascals et de préférence de l'ordre de 5.10⁵ à 60.10⁵ Pascals au moyen d'une presse à vis, de filtres presses ou avec tout autre équipement permettant d'exprimer un jus à partir de marc humide.

Eventuellement, on peut rediluer les marcs avec deux à quatre fois leur volume d'eau et les presser sur une presse identique à celle utilisée lors du premier pressage. Cette opération n'est pas indispensable mais permet d'augmenter le rendement d'extraction en pentoses. On peut également, afin d'augmenter les concentrations en pentoses, recycler tout ou partie le jus brut acide pour hydrolyser de nouveaux sons ou fibres de blé, ou encore de la paille de blé. Ce recyclage permet d'augmenter la teneur en matière sèche des jus et de diminuer de 30 % la consommation en acide nécessaire pour l'extraction des pentoses.

On peut également précipiter les impuretés contenues dans le jus de pentoses en présence de sels chélatants. On peut notamment utiliser des cations métalliques tels que l'hydroxyde d'aluminium, le sulfate d'aluminium, des cations alcalino-terreux tels que les hydroxydes de magnésium, de calcium, de strontium ou de baryum, les sulfates de magnésium, de calcium, de strontium ou de baryum, les chlorures de magnésium, de calcium, de strontium ou de baryum.

Pour mieux débarrasser le sirop de pentoses du marc solide et des impuretés précipitées, on peut éventuellement centrifuger le jus obtenu au moyen d'un équipement permettant d'obtenir des jus débarrassés d'insolubles, tels qu'une décanteuse horizontale, un filtre presse, un décanteur statique, des systèmes de filtration tangentielle, ou une centrifugeuse à assiettes auto-débourbeuse.

On peut éventuellement déminéraliser la solution de pentoses ou jus par chromatographie sur au moins une résine échangeuse d'ions, par chromatographie d'exclusion à basse pression, par électrodialyse ou encore par une combinaison des méthodes précédentes tout en laissant, de préférence, pour ne pas à avoir à ajouter ensuite de l'acide servant de catalyseur, au moins 0,02 équivalent H⁺ par mole de pentoses. En chromatographie, on réunit donc la fraction de pentoses à une partie de la fraction de sels. Sur résine d'échange d'ions, on passe de préférence successivement sur une résine échangeuse de cations et sur une résine échangeuse d'anions, la quantité de résine échangeuse d'anions pouvant être inférieure d'au moins 0,02 équivalents H+ à la stoechiométrie.

On peut également concentrer le sirop de pentoses par évaporation jusqu'à 30 à 95 % et plus particulièrement 60 à 80 % de matière sèche. Le sirop de pentoses débarrassé du marc solide présente une pureté en pentoses généralement comprise entre 50 et 95 % par rapport à la matière sèche.

Le troisième stade du procédé suivant l'invention consiste à mettre le sirop de pentoses débarrassé du marc en contact avec un alcool ayant de 6 à 22 atomes de carbone. L'alcool répond à la formule R¹OH, R¹ étant un radical linéaire ou ramifié alkyle de 6 à 22 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle. On préfère tout particulièrement les alcools gras ayant de 8 à 16 atomes de carbone ; et notamment, les mélanges d'octanol et de décanol, les mélanges de décanol et de dodécanol, les mélanges de dodécanol et de tétradécanol et les mélanges de dodécanol, de tétradécanol et d'hexadécanol. On utilise en général de 1 à 4 équivalents d'alcool par rapport au sucres. On préfère cependant dans un premier temps réaliser la glycosylation d'un alcool court par les pentoses du sirop. On réalise par la suite une transglycosylation par des alcools gras de formule R¹OH. L'alcool court répond à la formule R²OH, R² étant un radical linéaire alkyle ayant de 1 à 5 atomes de carbone. On préfère effectuer cette réaction de glycosylation en l'absence totale de solvants, mais on peut le cas échéant utiliser un solvant tel qu'un éther oxyde tel que le tétrahydrofurane, l'oxyde diéthylique, le 1,4-dioxane, l'oxyde isopropylique, le méthyl-tertiobutyl éther, l'éthyl-tertiobutyl éther ou le diglyme, un hydrocarbure halogéné tel que le chlorure de méthylène, le chloroforme, le 1,2-dichloroéthane, un solvant nitré tel que le nitrométhane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène. On préfère effectuer cette réaction entre 30 et 110°C pour prévenir le plus possible la formation de produit coloré, mais il est possible de l'effectuer jusqu'à 150°C. Dans le cas où le jus de pentoses a été complètement déminéralisé, il est nécessaire d'ajouter au milieu réactionnel 0,1 à 5 % en masse par rapport à la matière sèche du sirop d'un catalyseur acide. Cet acide pouvant être l'acide chlorydrique, l'acide sulfurique, l'acide phosphorique, un acide sulfonique tel que l'acide benzènesulfonique, l'acide paratoluènesulfonique, l'acide camphresulfonique, l'acide sulfosuccinique ou un sulfosuccinate d'alkyle tel que le sulfosuccinate de décyle ou de lauryle, les acides perhalohydriques, tels que par exemple l'acide perchlorique. Cette catalyse acide peut être également effectuée par 0,05 à 6 équivalents pondéraux d'une résine sulfonique sous sa forme H⁺, ou d'une résine acide. On préfère éviter la déminéralisation totale du jus de presse et par conséquent ne pas rajouter de catalyseur acide.

Pour recueillir le mélange de pentosides d'alkyle, le procédé consiste :
- à éliminer le solvant de réaction, s'il est présent,
- à neutraliser l'acidité du sirop de départ ou le catalyseur acide, puis à filtrer le sel obtenu. On effectue la neutralisation, par exemple, par un hydrogénocarbonate ou un carbonate de métal alcalin ou alcalino-terreux, notamment l'hydrogénocarbonate de sodium, par un hydroxyde de métal alcalin ou alcalino-terreux, notamment la soude,
- à purifier le produit souhaité :
   - soit par évaporation de l'alcool gras en excès sous vide compris entre 0,001 et 10 mbars à une température comprise entre 60 et 250°C,
   - soit par chromatographie sur colonne de gel de silice, d'alumine, de charbon actif ou sur résine échangeuse d'ions,
   - soit par cristallisation dans un solvant ou un mélange de solvants appropriés choisis parmi les éthers oxydes tel que le tétrahydrofurane, l'oxyde diéthylique, le 1,4-dioxane, l'oxyde isopropylique, le méthyl-tertiobutyl éther, l'éthyl-tertiobutyl éther ou le diglyme, un hydrocarbure halogéné tel que le chlorure de méthylène, le chloroforme, le 1,2-dichloroéthane, un solvant nitré tel que le nitrométhane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène,
   - soit par extractions sélectives par des solvants non miscibles à l'eau,
      Le produit isolé présente alors un pourcentage d'alcool gras résiduel compris entre 0 et 5% et de préférence entre 0 et 1%.
- éventuellement solubiliser le tensioactif dans l'eau pour obtenir une composition tensioactive présentant une matière sèche de 20 à 80 %,
- si besoin, décolorer cette solution en ajoutant à une température comprise entre 15 et 100 °C, 0,05 à 10 % et de préférence de 0,5 à 3 % de peroxyde d'hydrogène, de peroxodisulfates de métaux alcalins ou alcalino-terreux, de perborates, de persulfates, de perphosphates, de percarbonates, d'ozone ou encore de periodinates. On préfère le peroxyde d'hydrogène à 30 ou 50 %. L'agent de décoloration de la présente invention doit naturellement être compatible avec l'ensemble des ingrédients de la formulation finale et avec les applications des produits finis.
   En pratique, le procédé consiste à mélanger de la fibre ou du son désamylacé ou brut de granulométrie comprise entre 0,8 et 1,5 mm, ou encore de la paille de blé de granulométrie comprise entre 0,8 et 150 mm à au moins deux fois son volume d'eau contenant 0,01 à 15 % et plus particulièrement 1 à 10 % , par rapport à la matière sèche de la paille, du son ou d'une fibre de blé, d'acide. On préfère l'acide sulfurique, l'acide chlorhydrique ou l'acide sulfosuccinique,
- à homogénéiser, puis à cuire le milieu à une température comprise entre 20 et 150 °C, plus particulièrement entre 60 et 120 °C, pendant une durée de 1 à 60 minutes,
- dans le cas du traitement du son ou de la paille de blé, à presser le mélange à une pression de l'ordre de 2.10⁵ à 500.10⁵ Pascals et de préférence de l'ordre de 5.10⁵ à 60.10⁵ Pascals au moyen d'une presse à vis, de filtres presses ou avec tout autre équipement permettant d'exprimer un jus à partir de marc humide. Cette opération permet d'isoler un jus 1 et un marc 1,
- éventuellement à diluer les marcs 1 avec au moins trois fois leur volume d'eau et les presser sur une presse identique à celle utilisée lors du premier pressage. Cette opération n'est pas indispensable mais permet d'augmenter le rendement d'extraction en pentoses. On isole alors le jus 2 et le marc 2,
- à rassembler les jus des presses (jus 1 et jus 2)
- éventuellement à recycler une partie ou l'ensemble du jus brut acide 2 pour l'hydrolyse de nouveaux sons, pailles ou autre fibre de blé, et de préférence avec une quantité de jus brut acide similaire à la première dilution. Plusieurs recyclages à la suite sont possibles et permettent d'hydrolyser le son ou la fibre de blé, ou encore la paille sans apport d'eau supplémentaire, seule la quantité d'acide est ajustée,
- éventuellement précipiter les impuretés contenues dans le jus de pentoses en présence de sels chélatants. On peut notamment utiliser des cations métalliques tels que l'hydroxyde d'aluminium, le sulfate d'aluminium, des cations alcalino-terreux tels que les hydroxydes de magnésium, de calcium, de strontium ou de baryum, les sulfates de magnésium, de calcium, de strontium ou de baryum, les chlorures de magnésium, de calcium, de strontium ou de baryum.
- si nécessaire à centrifuger le jus obtenu au moyen d'un équipement permettant d'obtenir des jus débarassés d'insolubles, tels qu'une décanteuse horizontale, un filtre presse, un décanteur statique, des systèmes de filtration tangentielle, ou une centrifugeuse à assiettes auto-débourbeuse. On obtient alors le jus 3 dépourvu d'insolubles et une crème qui représente de 0,5 à 3 % du volume total avant séparation et une matière sèche de 4,5 % de la matière sèche initiale,
- éventuellement à déminéraliser le jus par chromatographie sur résine échangeuse d'ions, par chromatographie d'exclusion à basse pression de type acide, par électrodialyse ou encore par précipitation des anions contenus dans le jus en présence de sels chélatants,
- à concentrer le jus 3 par évaporation jusqu'à 20 à 80 % et plus particulièrement 60 à 80 % de matière sèche. Le sirop présente une pureté en pentoses généralement comprise entre 50 et 90 % par rapport à la matière sèche,
- on effectue la glycosylation par l'alcool.

Les mélanges de pentosides d'alkyle selon l'invention présentent des propriétés très intéressantes et trouvent notamment des applications dans les domaines de la détergence, de la cosmétique et de l'alimentaire.

Ces mélanges de pentosides d'alkyle sont de remarquables agents tensioactifs non-ioniques. Ils possèdent en particulier des propriétés de solubilisation, émulsionnantes, moussantes, mouillantes et dispersantes. Ils peuvent être utilisés comme détergents, notamment dans des compositions lessivielles et dans des compositions de lavage de la vaisselle, des sols et des vitres, comme adoucissants et comme adjuvants de détergents dans ces mêmes compositions aussi bien en poudre que liquide. Ils peuvent entrer également dans les compositions capillaires ou de dentifrice et être utiles en cosmétologie sous forme de pommades, de crèmes et de laits de beauté afin d'adoucir et de raffermir la peau en tant que tensioactif doux n'agressant ni la peau, ni les muqueuses. Leurs pouvoirs moussant et adoucissant peuvent être mis aussi à profit dans les compositions de bains moussants. Ils peuvent également entrer dans les formulations de produits alimentaires comme additifs d'huile et de graisse, dans les crèmes mayonnaises et des sauces. Dans l'industrie, ils peuvent servir d'agents pour effectuer des polymérisations en dispersion et en émulsion. Ce sont d'autre part des substances aptes à former des vésicules et présentent des propriétés de cristal liquide.

Parmi ces propriétés, on doit tout particulièrement mentionner celle permettant d'abaisser la tension superficielle notamment de l'eau, le pouvoir moussant, le pouvoir émulsionnant et le pouvoir détergent.

Parmi les dérivés de l'invention, on préfère pour leur bon pouvoir moussant ceux dans lesquels R¹ est alkyle et notamment alkyle ayant de 6 à 14 atomes de carbone et mieux encore de 8 à 12 et plus particulièrement 10 atomes de carbone dont le pouvoir moussant est supérieur aux produits classiques de référence. L'évaluation est réalisée selon la norme NFT 73404 qui consiste à faire s'écouler, selon un débit constant, 500 ml de solution de tensioactif dans une éprouvette de 1000 ml graduée, thermostatée et contenant 50 ml de la même solution. La quantité de mousse générée par l'écoulement est estimée volumétriquement juste après la fin de l'écoulement et ensuite la stabilité du volume de mousse est évaluée en fonction du temps pendant 20 minutes. Les mélanges de pentosides de décyl-dodécyle présentent un pouvoir moussant supérieur à ceux des tensioactifs les plus couramment rencontrés tels que le dodécylbenzènesulfonate de sodium (SDBS), le dodécyl sulfate de sodium (SDS), le lauryléthersulfate de sodium à 2 moles d'oxyde d'éthylène (LES) ou la dodécylbétaïne.

Avec les mélanges selon l'invention, le volume initial de mousse est de 530 ml alors qu'il est inférieur à 450 ml pour les autres. Leur pouvoir moussant est également supérieur à ceux des polyglucosides d'alkyle (APG). Par ailleurs, ces composés présentent une très bonne stabilité dans le temps de la mousse, puisque le pourcentage de perte est généralement inférieur à 5 % après 20 minutes.

Les mélanges de pentosides d'alkyle selon l'invention abaissent très efficacement la tension superficielle de l'eau. Cette propriété a été déterminée par une technique usuelle de tensiométrie, en utilisant un tensiomètre Schlumberger Tensimat n° 3. La mesure est effectuée à 25°C. Le mobile de mesure est une lame de platine rectangulaire (25 mm x 5 mm). Quand R¹ a moins de 12 atomes de carbone, cet abaissement de la tension superficielle de l'eau est supérieur à celui observé avec les produits connus (SDBS, SDS, LES, APG). La mise en émulsion des salissures portées par du linge est ainsi rendue plus facile.

Pour leur pouvoir émulsionnant, on préfère tout particulièrement ceux dans lesquels R¹ est alkyle et notamment alkyle ayant de 8 à 22 atomes de carbone et mieux encore de 14 à 20.

Il convient enfin de souligner que les pentosides d'alkyle selon l'invention sont entièrement biodégradables et sans danger pour l'environnement, tout en présentant une bonne stabilité.

La présente invention a donc également pour objet un procédé pour donner de la tensioactivité à une composition caractérisée en ce qui consiste à lui incorporer de 0,1 à 60 % en poids d'un dérivé ou d'un mélange de dérivés suivant l'invention.

Les compositions selon l'invention peuvent se présenter sous différentes formes, notamment sous forme de détergents en poudre ou liquide, de lotions, moussantes ou non moussantes, d'émulsions de consistance liquide ou semi-liquide telles que des laits obtenus par dispersion d'une phase grasse dans une phase aqueuse ou inversement, de suspensions ou d'émulsions de consistance molle du type crèmes ou pommades, de gels ou encore de préparations solides telles que des sticks, des pains de nettoyage, des tampons imprégnés ou encore sous forme de masques hydratants.

Comme véhicule des compositions selon l'invention, on peut utiliser de l'eau, des solvants organiques compatibles avec une application topique tels que l'acétone, l'alcool isopropylique, les triglycérides d'acides gras en C₆-C₂₄, les éthers de glycol tels que les éthers d'alkyle inférieur de mono ou dialkylène glycol, dont le radical alkylène a de 2 à 4 atomes de carbone.

On peut également utiliser comme solvant des esters de polyalkylèneglycol et d'acide à chaîne courte en C₁-C₄, ou encore des silicones volatiles.

Les compositions selon l'invention peuvent également contenir des corps gras tels que des huiles naturelles ou synthétiques.

Les compositions selon l'invention peuvent également contenir des agents épaississants ou gélifiants tels que la cellulose ou des dérivés de cellulose. Les agents épaississants peuvent être également des polymères acryliques, des alginates, des gommes telles que la gomme de xanthane, de guar, de caroube ou la gomme arabique ou encore des polyéthylène glycols, des bentonites et des montmorillonites.

Les compositions selon l'invention peuvent également contenir des matières actives comme des agents hydratants ainsi que des adjuvants tels que des agents antioxydants, des agents conservateurs, des parfums, des colorants.

Les compositions selon l'invention peuvent également se présenter sous forme de solutions ou de dispersions contenant les pentosides d'alkyle sous forme vésiculaire, les vésicules pouvant alors servir d'agents d'encapsulation pour des ingrédients actifs lipophiles et/ou hydrophiles.

Une composition détergente en poudre suivant l'invention comprend de 0,1 à 60 %, et de préférence de 10 à 30 % en poids d'une base détergente et de 99,9 à 40%, et de préférence de 90 à 70 % en poids d'adjuvants.

La base détergente peut être un dérivé ou un mélange de dérivés selon l'invention. Elle peut également être un mélange de un ou plusieurs dérivés selon l'invention avec un ou plusieurs tensioactifs classiques dans le domaine ; ces tensioactifs pouvant être anioniques, non ioniques, cationiques ou amphotères. La proportion de tensioactifs selon l'invention représente de 1 à 100 %, en poids, et de préférence de 50 à 100 % de l'ensemble de la charge en tensioactifs.

La composition détergente en poudre est utilisée à une concentration de 1 à 20 g/l, et de préférence entre 1 et 6 g/l. Le lavage est effectué dans une machine conventionnelle, entre 20 et 80°C, et de préférence entre 20 et 60°C, durant une période de 10 à 60 minutes.

Une composition détergente liquide suivant l'invention comprend de 0,1 à 60 %, et de préférence de 10 à 60 % en poids d'une base détergente de 99,9 à 40 % et de préférence de 90 à 70 % en poids d'adjuvants.

La base détergente liquide peut être un dérivé ou un mélange de dérivés selon l'invention. Elle peut également être un mélange de un ou plusieurs dérivés selon l'invention avec un ou plusieurs tensioactifs classiques dans le domaine.

La composition détergente liquide selon l'invention est utilisée en solution aqueuse à une concentration de 6 à 12 g/l, et à des températures de 40 à 70°C.

Peuvent être présents dans des compositions détergentes, des additifs du type de ceux décrits ci-après :
- AGENTS TENSIOACTIFS autres, en quantités correspondant à environ 3-40% en poids par rapport à la composition détergente, agents tensioactifs tels que
   agents tensioactifs anioniques. les alkylesters sulfonates de formule R-CH(SO₃M)-COOR', où R représente un radical alkyle en C₈-₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpiperidinium...) ou dérivé d'une alcanolamine (monoéthanolamine, diéthanolamine, triéthanolamine...). On peut citer tout particulièrement les méthyl ester sulfonates dont le radical R est en C14-C16 ;
   . les alkylsulfates de formule ROSO₃M, où R représente un radical alkyle ou hydroxyalkyle en C₁₀-C₂₄, de préférence en C₁₂-C₂₀ et tout particulièrement en C₁₂-C₁₈, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;
   . les alkylamides sulfates de formule RCONHR'OSO₃M où R représente un radical alkyle en C2-C22, de préférence en C6-C20, R' un radical alkyle en C2-C3, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
   . les alkyl-D-galactosides uronates de formule 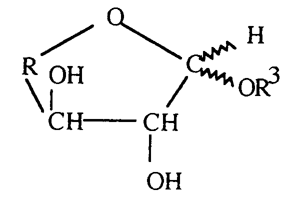 R³ étant un radical linéaire ou ramifié alkyle de 6 à 22 atomes de carbone et de préférence de 8 à 16 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle,
      R étant

      〉 CH-CH (OH)-CO₂R⁴

      ou 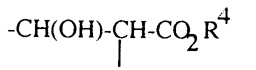 dont le carbone portant le groupe hydroxy n'est pas relié à l'atome d'oxygène endocyclique. R⁴ étant l'hydrogène, un atome de métal alcalin, de métal alcalino-terreux, un groupe ammonium quaternaire ;
   . les sels d'acides gras saturés ou insaturés en C₈-C₂₄, de préférence en C₁₄-C₂₀, les alkylbenzènesulfonates en C₉-C₂₀, les alkylsulfonates primaires ou secondaires en C₈-C₂₂, les alkylglycérol sulfonates, les acides polycarboxyliques sulfonés décrits dans GB-A-1 082 179, les sulfonates de paraffine, les N-acyl N-alkyltaurates, les alkylphosphates, les alkyliséthionates, les alkylsuccinamates, les alkylsulfosuccinates, les monoesters ou diesters de sulfosuccinates, les N-acyl sarcosinates, les sulfates d'alkylglycosides, les polyéthoxycarboxylates, le cation étant un métal alcalin (sodium, potassium, lithium), un reste ammonium substitué ou non substitué (méthyl-, diméthyl-, triméthyl-, tétraméthylammonium, diméthylpiperidinium...) ou dérivé d'une alcanolamine (monoéthanolamine, diéthanolamine, triéthanolamine...) ;
      agents tensioactifs non-ioniques
   . les alkylphénols polyoxyalkylénés (polyéthoxyéthylénés, polyoxypropylénés, polyoxybutylénés) dont le substituant alkyle est en C₆-C₁₂ et contenant de 5 à 25 motifs oxyalkylènes ; à titre d'exemple, on peut citer les TRITON X-45, X-114, X-100 ou X-102 commercialisés par Rohm & Haas Cy.;
   . les glucosamides, glucamides ;
   . les glycérolamides dérivés de N-alkylamines (US-A-5,223,179 et FR-A-1,585,966)
   . les alcools aliphatiques en C₈-C₂₂ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy.
   . les produits résultant de la condensation de l'oxyde d'éthylène avec un composé hydrophobe résultant de la condensation de l'oxyde de propylène avec le propylène glycol, tels les PLURONIC commercialisés par BASF ;
   . les produits résultant de la condensation de l'oxyde d'éthylène le composé résultant de la condensation de l'oxyde de propylène avec l'éthylènediamine, tels les TETRONIC commercialisés par BASF ;
   . les oxydes d'amines tels que les oxydes d'alkyl C₁₀-C₁₈ diméthylamimes, les oxydes d'alkoxy C8-C22 éthyl dihydroxy éthylamines :
   . les alkylpolyglycosides décrits dans US-A-4 565 647 ; les amides d'acides gras en C8-C20
   . les acides gras éthoxylés
   . les amides gras éthoxylés
   . les amines éthoxylées
      agents tensioactifs cationiques
   . les halogénures d'alkyldiméthylammonium
      agents tensioactifs amphotères et zwitterioniques
   . les alkyldiméthylbétaïnes, les alkylamidopropyldiméthylbétaïnes, les alkyltriméthylsulfobétaïnes, les produits de condensation d'acides gras et d'hydrolysats de protéines.
- AGENTS BUILDERS, en quantités correspondant à environ 5-50%, de préférence à environ 5-30% en poids pour les formules détergentes liquides, ou à environ 10-80%, de préférence 15-50% en poids pour les formules détergentes en poudres, agents builders tels que
   builders inorganiques
   . les polyphosphates (tripolyphosphates, pyrophosphates, orthophosphates, hexamétaphosphates) de métaux alcalins, d'ammonium ou d'alcanolamines
   . les tétraborates ou les précurseurs de borates
   . les silicates, en particulier ceux présentant un rapport SiO₂/Na₂O de l'ordre de 1,6/1 à 3,2/1 et les silicates lamellaires décrits dans US-A-4 664 839
   . les carbonates (bicarbonates, sesquicarbonates) alcalins ou alcalino-terreux
   . les cogranulés de silicates hydratés de métaux alcalins et de carbonates de métaux alcalins (sodium ou de potassium) riches en atomes de silicium sous forme Q2 ou Q3, décrits dans EP-A-488 868
   . les aminosilicates cristallins ou amorphes de métaux alcalins (sodium, potassium) ou d'ammonium, tels que les zéolithes A, P, X... ; la zéolithe A de taille de particules de l'ordre de 0,1-10 micromètres est préférée
      builders organiques
   . les polyphosphonates hydrosolubles (éthane 1-hydroxy-1, 1-diphosphonates, sels de méthylène diphosphonates...)
   . les sels hydrosolubles de polymères ou de copolymères carboxyliques ou leurs sels hydrosolubles tels que
      . les éthers polycarboxylates (acide oxydisuccinique et ses sels, tartrate monosuccinic acide et ses sels, tartrate disuccinic acide et ses sels)
      . les éthers hydroxypolycarboxylates
      . l'acide citrique et ses sels, l'acide mellitique, l'acide succinique et leurs sels
      . les sels d'acides polyacétiques (éthylènediaminetétraacétates, nitrilotriacétates, N-(2 hydroxyéthyl)-nitrilodiacétates)
      . les acides alkyl C5-C20 succiniques et leurs sels (2-dodécénylsuccinates, lauryl succinates,)
      . les esters polyacétals carboxyliques
      . l'acide polyaspartique, l'acide polyglutamique et leurs sels
   . les polyimides dérivés de la polycondensation de l'acide aspartique et/ou de l'acide glutamique
   . les dérivés polycarboxyméthylés de l'acide glutamique ou d'autres acides aminés

On peut utiliser ces builders en mélange selon différentes proportions. Le rapport de la charge de l'ensemble des builders sur celle de l'ensemble de la base tensioactive est compris entre 1 et 4, et de préférence entre 2 et 3.
- Les AZURANTS OPTIQUES sont ceux utilisés classiquement dans le domaine, notamment l'acide stilbène disulfonique ou les dérivés du bis-(styryl)biphényle.
- AGENTS DE BLANCHIEMENT, en quantités d'environ 0,1-20% de préférence environ 1-10% de poids, éventuellement associés à des ACTIVATEURS DE BLANCHIEMENT, en quantités d'environ 0,1-60%, de préférence d'environ 0,5-40% en poids, agents et activateurs tels que :
   agents de blanchiement
   . les perborates tels que le perborate de sodium monohydraté ou tétrahydraté
   . les composés peroxygénés tels que le carbonate de sodium peroxyhydraté, le pyrophosphate peroxyhydraté, l'urée peroxyhydratée, le peroxyde de sodium, le persulfate de sodium de préférence associés à un activateur de blanchiement générant in situ dans le milieu lessiviel, un peroxyacide carboxylique ; parmi ces activateurs, on peut mentionner, la tétraacétyléthylène diamine, la tétraacétylméthylène diamine, le tétraacétyl glycoluryl, le p-acétoxybenzène sulfonate de sodium, le pentaacétyl glucose, l'octaacétyl lactose...
   . les acides percarboxyliques et leurs sels (appelés "percarbonates") tels que le monoperoxyphtalate de magnésium hexahydraté, le métachloroperbenzoate de magnésium, l'acide 4-nonylamine-4-oxoperoxybutyrique, l'acide 6-nonylamine-6-oxoperoxycaproïque, l'acide diperoxydodécanedioïque, le nonylamide de l'acide peroxysuccinique, l'acide décyldiperoxysuccinique.
      Ces agents peuvent être associés à au moins un des agents anti-salissures ou anti-redéposition mentionnés ci-après.
      Peuvent également être mentionnés des agents de blanchiement non oxygénés, agissant par photoactivation en présence d'oxygène, agents tels que les phtalocyanines d'aluminium et/ou de zinc sulfonées
      AGENTS ANTI-SALISSURES, en quantités de l'ordre de 0,01-10%, de préférence environ 0,1-5%, et tout particulièrement de l'ordre de 0,2-3% en poids, agents tels que
   . les dérivés cellulosiques tels que les hydroxyéthers de cellulose, la méthylcellulose, l'éthylcellulose, l'hydroxypropyl méthylcellulose, l'hydroxybutyl méthylcellulose
   . les polyvinylesters greffés sur des troncs polyalkylènes tels que les polyvinylacétates greffés sur des troncs polyoxyéthylènes (EP-A-219 048)
   . les alcools polyvinyliques
   . les copolymères polyesters à base de motifs ethylène téréphtalate et/ou propylène téréphtalate et polyoxyéthylène téréphtalate, avec un rapport molaire (nombre de motifs) ethylène téréphtalate et/ou propylène téréphalate / (nombre de motifs) polyoxyéthylène téréphtalate de l'ordre de 1/10 à 10/1, de préférence de l'ordre de 1/1 à 9/1, les polyoxyéthylène téréphtalates présentant des unités polyoxyéthylène ayant un poids moléculaire de l'ordre de 300 à 5000, de préférence de l'ordre de 600 à 5000 (US-A-3 959 230, US-A-3 893 929, US-A4-116 896, US-A-4 702 857, US-A-4 770 666) ;
   . les oligomères polyesters sulfonés obtenus par sulfonation d'un oligomère dérivé de l'alcool allylique éthoxylé, du diméthyltéréphtalate et du 1,2 propylène diol, présentant de 1 à 4 groupes sulfonés (US-A-4 968 451)
   . les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A- 702 857) ou des groupes anioniques sulfopolyéthoxy (US-A-4 721 580), sulfoaroyles (US-A-4 877 896)
   . les polyesters-polyuréthanes obtenus par réaction d'un polyester de masse moléculaire en nombre de 300-4000 obtenus à partir d'acide adipique et/ou d'acide téréphtalique et/ou d'acide sulfoisophtalique et d'un diol de masse inférieure à 300, sur un prépolymère à groupements isocyanates terminaux obtenus à partir d'un polyoxyéthylène glycol de masse moléculaire de 600-4000 et d'un diisocyanate (FR-A-2 334 698)
- AGENTS ANTI-REDEPOSITION, en quantité d'environ 0,01-10% en poids pour une composition détergente en poudre, d'environ 0,01-5% en poids pour une composition détergente liquide, agents tels que
   . les monoamines ou polyamines éthoxylées, les polymères d'amines éthoxylées (US-A-4 597 898, EP-A-11 984)
   . la carboxyméthylcellulose
   . les oligomères polyesters sulfonés obtenus par condensation de l'acide isophtalique, du sulfosuccinate de diméthyle et de diéthylène glycol (FR-A-2 236 926)
   . les polyvinylpyrrolidones
- AGENTS CHELATANTS du fer et du magnésium, en quantités de l'ordre de 0,1-10% de préférence de l'ordre de 0,1-3% en poids, agents tels que
   . les aminocarboxylates tels que les nitrilotriacétates, les éthylènediaminetétraacétates, hydroxyéthyléthylènediaminetriacétates
   . les aminophosphonates tels que les nitrilotris-(méthylène phosphonates)
   . les composés aromatiques polyfonctionnels tels que les dihydroxydisulfobenzènes
- AGENTS DISPERSANTS POLYMERIQUES, en quantité de l'ordre de 0,1-7% en poids, pour contrôler la dureté en calcium et magnésium, agents tels que
   . les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acrylique, acide ou anhydride maléique, acide fumarique, acide itaconique, acide aconitique, acide mésaconique, acide citraconique, acide méthylènemalonique, et tout particulièrement les polyacrylates de masse moléculaire de l'ordre de 2000 à 10000 (US-A-3 308 067), les copolymères d'acide acrylique et d'anhydride maléique de masse moléculaire de l'ordre de 5 000 à 75 000 (EP-A-66 915)
   . les polyéthylèneglycols de masse moléculaire de l'ordre de 1000 à 50 000
- AGENTS DE FLUORESCENCE (BRIGHTENERS), en quantité d'environ 0,05-1,2% en poids, agents tels que les dérivés de stilbène, pyrazoline, coumarine, acide fumarique, acide cinnamique, azoles, méthinecyanines, thiophènes... ("The production and application of fluorescent brightening agents" - M. Zahradnik, publié par John Wiley & Sons, New York-1982-)
- AGENTS SUPPRESSEURS DE MOUSSES, en quantité pouvant aller jusqu'à 5% en poids, agents tels que
   . les acides gras monocarboxyliques en C10-C24 ou leurs sels alcalins, d'ammonium ou alcanolamines, les triglycérides d'acides gras
   . les hydrocarbures saturés ou insaturés aliphatiques, alicycliques, aromatiques ou hétérocycliques, tels que les paraffines, les cires
   . les N-alkylaminotriazines
   . les monostéarylphosphates, les monostéaryl alcool phosphates
   . les huiles ou résines polyorganosiloxanes éventuellement combinées avec des particules de silice
- AGENTS ADOUCISSANTS, en quantité d'environ 0,5-10% en poids, agents tels que les argiles
- ENZYMES en quantité pouvant aller jusqu'à 5 mg en poids, de préférence de l'ordre de 0,05-3mg d'enzyme active/g de composition détergente, enzymes telles que
   . les protéases, amylases, lipases, cellulases, peroxydases (US-A-3 553 139, US-A-4 101 457, US-A-4 507 219, US-A-4 261 868)
- AUTRES ADDITIFS tels que
   . des alcools (méthanol, éthanol, propanol, isopropanol, propanediol, éthylène glycol, glycérine)
   . des agents tampons
   . des parfums
   . des pigments

L'invention a également pour objet l'utilisation des dérivés selon l'invention pour obtenir une compositon détergente pour le lavage de la vaisselle, et pour les usages ménagers. Cette composition comprend de 1 à 50 % en poids et de préférence de 5 à 30 %, du mélange selon l'invention et 99 à 50 % en poids, et de préférence 95 à 70 % d'adjuvants ou d'autres tensioactifs.
Parmi les agents tensioactifs anioniques pouvant être présents, on peut notamment citer les alkylsulfates, les alkyléthersulfates, les alkylsulfonates, les alkylbenzènesulfonates, les savons, les alkyléthercarboxylates, les N-acylsarcosinates, les alkyliséthionates, les N-acyl N-alkyltaurates, les alkylphosphates, les alkylsulfosuccinates, les alkylsulfosuccinamates, les dérivés sulfonés d'acides gras...On peut également utiliser les alkyl-D-galactosides uronates de formule 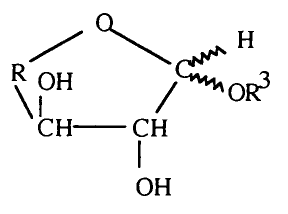 R³ étant un radical linéaire ou ramifié alkyle de 6 à 22 atomes de carbone et de préférence de 8 à 16 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle,
R étant

〉CH-CH (OH)-CO₂R⁴

ou 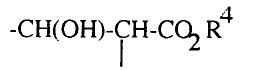 dont le carbone portant le groupe hydroxy n'est pas relié à l'atome d'oxygène endocyclique. R⁴ étant l'hydrogène, un atome de métal alcalin, de métal alcalino-terreux, un groupe ammonium quaternaire ;
Parmi les agents tensioactifs non-ioniques pouvant être présents, on peut notamment citer les oxydes d'amines, les alkylglucamides, les alkylpolyglycosides, les dérivés oxyalkylénés d'alcools gras comme les PLANTAREN® commercialisés par HENKEL...

A côté des tensioactifs non-ioniques de la présente invention, peuvent être présents dans les compositions pour liquides de lavage de la vaisselle à la main, d'autres additifs ou tensioactifs tels que :
. des agents tensioactifs amphotères et zwitterioniques comme les alkyldiméthylbétaïnes, les alkylamidopropylbétaïnes, les alkylsultaines, les produits de condensation d'acides gras sur les protéines ou les hydrolysats de protéines, les dérivés amphotères des alkylpolyamines comme l'AMPHIONIC XL® commercialisé par RHONE-POULENC, AMPHOLAC 7T/X® et AMPHOLAC 7C/X® commercialisés par BEROL NOBEL
. des agents bactéricides ou désinfectants comme le triclosan
. des polymères cationiques synthétiques comme le MIRAPOL A550®, le MIRAPOL A15® commercialisés par RHONE-POULENC, le MERQUAT 550® commercialisé par CALGON...
. les polymères utilisés pour contrôler la viscosité du mélange et/ou la stabilité des mousses formées à l'utilisation, comme les dérivés de cellulose ou de guar (carboxyméthylcellulose, hydroxyéthylcellulose, hydroxypropylguar, carboxyméthylguar, carboxyméthylhydroxypropylguar...)
. des agents hydrotropes, comme les alcools courts en C2-C8, en particulier l'éthanol, les diols et glycols comme le diéthylène glycol, dipropylèneglycol,...
. des agents hydratants ou humectants pour la peau comme le glycérol, l'urée ou des agents protecteurs de la peau, comme les protéines ou hydrolysats de protéines, les polymères cationiques, comme les dérivés cationiques du guar (JAGUAR C13S®, JAGUAR C162®, HICARE 1000® commercialisés par RHONE-POULENC),
. des colorants, des parfums, des conservateurs...

Les compositions selon l'invention sont agréables au toucher et se rincent facilement.

L'invention a aussi pour objet une composition cosmétique comprenant de 0,1 à 50 % et de préférence de 5 à 35 % en poids de substance tensioactive et de 99,9 à 50 % et de préférence de 95 à 65 % en poids d'adjuvant ou de principe actif, caractérisée en ce que 0,5 à 100 % des substances tensioactives sont au moins un mélange de l'invention.
Les tensioactifs non-ioniques, qui font l'objet de cette invention, peuvent être utilisés plus particulièrement dans des formulations de lotions, laits de toilettes, compositions démaquillantes, crèmes de soins ou crèmes ou lotions de protection contre le soleil et le rayonnement ultra-violet, mousses, gels coiffants ou toute formulation utilisée pour le coiffage ou pour faciliter le peignage des cheveux, shampooings pour cheveux ou le corps, gels de nettoyage du visage ou du corps, savons liquides, compositions moussantes pour le bain, formulations utilisées pour le nettoyage des dents ou de la cavité buccale comme les bains de bouche ou les dentifrices. Les tensioactifs non-ioniques de la présente invention peuvent aussi être utilisés seuls ou en association dans la formulation de savons ou de pains de toilette.

Les compositions cosmétiques contiennent généralement des agents tensioactifs qui servent à disperser, émulsionner, solubiliser, stabiliser divers composés utilisés pour leurs principes actifs, leurs propriétés émollientes ou humectantes. Parmi les principes actifs, on peut citer les enzymes, les vitamines, les anti-oxydants, les anti-microbiens, les anti-pelliculaires,...
Parmi les agents humectants, on peut citer le glycérol, le sorbitol, l'urée, le collagène, la gélatine, l'aloe vera, l'acide hyaluronique,...
Les émollients sont généralement choisis parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin...) ou les huiles d'origine animale (suif, huiles de poisson,...), des dérivés de ces huiles comme les huiles hydrogénées,les huiles synthétiques comme les poly-α-oléfines, les dérivés de la lanoline, les huiles minérales ou les huiles paraffiniques, le perhydrosqualane, le squalène, les diols comme le 1,2-propanediol, le 1,3-butanediol, l'alcool cétylique, l'alcool stéarylique, l'alcool oléique, les polyéthylèneglycols ou polypropylèneglycols, les esters gras comme le palmitate d'isopropyl, le cocoate d'éthyl-2-hexyl, le myristyl myristate, les esters de l'acide lactique, l'acide stéarique, l'acide béhennique, l'acide isostéarique, les huiles silicones regroupant les polydiméthylsiloxanes cycliques, les polydiméthylsiloxanes α-ω hydroxylées, les polydiméthylsiloxanes α-ω triméthylsilyllés, les polyorganosiloxanes comme les polyalkylméthylsiloxanes, les polyméthylphénylsiloxanes, les polydiphénylsiloxanes, les dérivés aminés des silicones, les cires silicones, les silicones copolyéthers (comme l'huile SILBIONE 70646® commercialisée par la société RHONE-POULENC ou DC 190® commercialisée par DOW CORNING) ou les dérivés mixtes de silicones comme les copolymères mixtes polyalkylméthylsiloxanes-silicones copolyéthers.
A ces composés, on peut ajouter des poudres ou des particules minérales comme du carbonate de calcium, des oxydes minéraux sous forme de poudre ou sous forme colloïdale (particules de taille inférieure ou de l'ordre de un micromètre, parfois de quelques dizaines de nanomètres) comme du dioxyde de titane, de la silice, des sels d'aluminium utilisés généralement comme antitranspirants, du kaolin, du talc, des argiles et leurs dérivés,...
Des agents conservateurs comme les méthyl, éthyl, propyl et butyl esters de l'acide p-hydroxybenzoïque, le benzoate de sodium, le GERMABEN® ou tout agent chimique évitant la prolifération bactérienne ou des moisissures et utilisés traditionnellement dans des compositions cosmétiques sont généralement introduits dans ces compositions à hauteur de 0,01 à 3% en poids.
Alternativement à ces agents chimiques, on peut parfois utiliser des agents modifiant l'activité de l'eau et augmentant fortement la pression osmotique comme les carbohydrates ou des sels.
Pour protéger la peau ou les cheveux des agressions du soleil et des rayons UV, on peut ajouter à ces formulations des filtres solaires qui sont, soit des composés chimiques absorbant fortement le rayonnement UV comme les composés autorisés dans la directive Européenne N° 76/768/CEE, ses annexes et les modifications ultérieures de cette directive, soit le dioxyde de titane ou les oxydes de cérium sous forme de poudre ou de particules colloïdales.
Des parfums, des colorants ou des pigments peuvent également être ajoutés.
Peuvent aussi être présents des polymères viscosants ou gélifiants (comme les polyacrylates réticulés CARBOPOL® commercialisés par GOODRICH-, les dérivés de la cellulose comme l'hydroxypropylcellulose, la carboxyméthylcellulose, les guars et leurs dérivés, la caroube, la gomme de tara ou de cassia, la gomme xanthane, les alginates, les carraghénannes, les dérivés de la chitine comme le chitosan...

Un dentifrice peut contenir, outre des agents abrasifs comme de la silice ou du carbonate de calcium (utilisés à 5 à 25% en poids du total de la composition), de 0,1 à 5% en poids desdits tensioactifs de l'invention, pris seuls ou en mélange avec les tensioactifs usuels tels que des tensioactifs anioniques, non ioniques ou zwitterioniques comme les alkylbétaïnes ou alkylamidopropylbétaïnes ou les amphotères ou des associations de ces composés. Ces agents tensioactifs sont utilisés comme agents moussants lors de l'utilisation de la composition, ainsi que pour leur pouvoir nettoyant, désinfectant. Parfois, ils sont utilisés spécifiquement pour une action "thérapeutique" comme les alkyl sarcosinates qui protègent les dents en inhibant l'activité enzymatique des bactéries responsables des infections des dents ou des gencives. Les compositions de dentifrices comportent aussi de 5 à 85% d'agents dits humectants comme le glycérol, le sorbitol, les polyéthylèneglycols, le lactilol, le xylitol.
Le comportement rhéologique de la pâte dentaire, c'est-à-dire sa viscosité, sa tenue sur la brosse, la facilité de son extrusion à la sortie du tube ou du dispenseur, le caractère non filant de la pâte est contrôlé par des agents épaississants comme certaines silices utilisées à cet effet (TIXOSIL 43® commercialisées par RHONE-POULENC) et/ou des polymères utilisés seuls ou en association comme la gomme xanthane, la gomme guar, les dérivés de la cellulose (carboxyméthylcellulose par exemple), des polyacrylates réticulés comme les CARBOPOL® distribués par GOODRICH, des alginates ou des carraghénannes, de la VISCARIN®. Le total des agents épaississants représente de 0,1 à 15% en poids de la composition d'un dentifrice.
On retrouve aussi généralement associés à ces divers constituants des agents thérapeutiques comme certains sels de fluor ou de potassium, des arômes, des agents édulcorants et de l'eau.

Les tensioactifs de cette invention peuvent être aussi utilisés dans des formulations de pains de toilette appelées savonnettes ou savons.

Les compositions classiques de pains de toilette comprennent généralement des sels d'acide gras utilisés en association avec des tensioactifs de l'invention et éventuellement des tensioactifs autres que les sels d'acides gras ou les acides gras eux-mêmes. Ces compositions peuvent même ne contenir aucun acide gras ou sel d'acide gras et leurs formulations reposent alors sur d'autres tensioactifs comme par exemple les alkyliséthionates de sodium en C₈-C₂₂ ou les alkylsulfates de sodium en C₈-C₂₂ ou encore les alkyl-D-galactosides uronates de formule 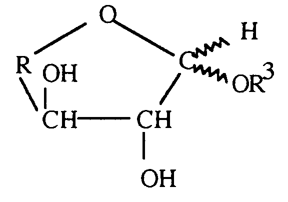 R³ étant un radical linéaire ou ramifié alkyle de 6 à 22 atomes de carbone et de préférence de 8 à 16 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle,
R étant

〉CH-CH (OH)-CO₂R⁴

ou 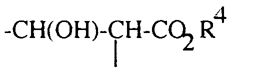 dont le carbone portant le groupe hydroxy n'est pas relié à l'atome d'oxygène endocyclique. R⁴ étant l'hydrogène, un atome de métal alcalin, de métal alcalino-terreux, un groupe ammonium quaternaire ;

A ces compositions on peut aussi ajouter différents constituants utiles pour diminuer l'irritation ou l'aggression de la peau comme les sels de métaux alcalins ou les iséthionates ou pour favoriser l'hydratation de la peau comme certains carbohydrates (glycérol, sorbitol par exemple), des polyethylènes glycols ou polypropylènes glycols, des dérivés alcoxylés des sucres ou de leurs dérivés (méthyl glucose par exemple), des polymères hydrosolubles ou hydrodispersables comme le collagène ou certains dérivés non allergisants de protéines animales ou végétales (hydrolysats de protéines de blé par exemple), des hydrocolloïdes naturels (gomme de guar, de caroube, de tara...) ou issus de procédés de fermentation comme la gomme xanthane et les dérivés de ces polycarbohydrates comme les celluloses modifiées (par exemple Hydroxyéthylcellulose, carboxyméthylcellulose, celluloses cationiques comme les POLYMER JR® commercialisés par la société UNION CARBIDE), les dérivés du guar ou de la caroube comme leurs dérivés cationiques (JAGUAR C13S®, JAGUAR C162® commercialisés par RHONE-POULENC) ou des dérivés non-ioniques (par exemple hydroxypropylguar), les dérivés anioniques (carboxyméthylguar) ou les dérivés mixtes non-ioniques /anioniques comme les carboxy-hydroxypropyl-guars ou non-ioniques/cationiques. On peut aussi ajouter alternativement ou en association des polymères synthétiques comme les polyacrylates ou des polymères cationiques synthétiques, connus sous le nom générique CTFA de "Polyquaternium", par exemple des polymères MIRAPOL A15® ou MIRAPOL 550® de la société RHONE-POULENC.
On peut aussi avantageusement ajouter à ces compositions des agents séquestrants des métaux, plus particulièrement ceux séquestrants du calcium comme les ions citrates ou des agents émollients comme les silicones ou des huiles ou corps gras utilisés à ce propos dans l'industrie cosmétique (huiles minérales, esters d'acides gras, triglycérides, silicones,...). A ces ingrédients on rajoute généralement un ou des parfums, des colorants et/ou des agents opacifiants comme des pigments (particules d'oxyde de titane). On peut aussi incorporer dans la composition des agents bactéricides ou fongicides afin d'améliorer la désinfection de la peau.
Dans un pain de toilette dont la formulation est constituée principalement de savons d'acides gras monocarboxyliques (sels de sodium, potassium, mono-, di- ou tri-éthanolammonium) ; les teneurs en savons d'acides gras sont généralement de plus de 25% en poids de la formulation, généralement de 30 à 95% en poids.
Dans un pain de toilette dont la formulation repose sur d'autres constituants principaux que les savons d'acides gras, on trouve dans la formulation de 0 à 50% en poids, préférentiellement de 1 à 40% en poids de ces savons d'acides gras.
Ces compositions de pains de toilette peuvent aussi contenir de 0 à 95%, de préférence de 0 à 60% d'agents tensioactifs autres que des savons, notamment les C8-C22 alkyl ou alkényl iséthionates, de même que les alkyléthersulfates, les alkylbétaïnes, les alkylamidopropylbétaïnes, les alkylamphoacétates, -diacétates, -propionates ou -dipropionates utilisés pour diminuer l'irritation pouvant être provoquée par les autres agents tensioactifs, principalement les agents tensioactifs anioniques.
De 1 à 15% d'acides gras libres en C8-C22 peuvent aussi être introduits dans les compositions de savon en tant qu'agents surgraissants ou pour modifier l'aspect et le caractère crèmeux de la mousse lors du lavage.
On peut aussi retrouver dans ces compositions des cires comme des cires paraffiniques, des cires naturelles comme la cire d'abeille ou l'ozokérite ou des cires silicones. Ces cires sont avantageusement utilisées pour améliorer l'aspect, la tenue, la processabilité et la conservation au stockage des pains de toilette.

La composition cosmétique peut être un bain moussant contenant de 5 à 40 % d'une base détergente, elle-même constituée de plus de 50 % de dérivés ou mélange de dérivés selon l'invention, et d'adjuvants. Les autres constituants de la base détergente sont les composés classiques dans le domaine cités précédemment.

La composition cosmétique peut être un gel douche contenant 5 à 35 % d'une base détergente, elle-même constituée d'au moins 50 % de dérivés, ou mélange de dérivés suivant l'invention et d'adjuvants.

La composition cosmétique peut être un savon liquide doux, contenant 5 à 30 % en poids, et de préférence 5 à 20 %, d'un dérivé selon l'invention et 95 à 70 % en poids, et de préférence 95 à 80 % en poids d'excipients. Ces excipients peuvent être également des tensioactifs autres que ceux de l'invention.

La composition cosmétique peut, notamment par incorporation de dérivés selon l'invention, notamment qui forme une structure de type gel pour des concentrations en eau supérieures à 60 %, et de préférence supérieures à 90 %, être une crème de soins, pour le visage notamment.

La composition cosmétique peut être un shampooing, notamment un shampooing doux à usage fréquent. Il est composé de 5 à 35 % en poids d'une base détergente dont, de préférence, 10 à 75 % est constitué par un dérivé ou un mélange de dérivés selon l'invention, et de 95 à 65 % d'adjuvants.

Les shampooings, et d'une manière plus générale les compositions détergentes pour l'usage personnel peuvent contenir, outre les tensioactifs non-ioniques de l'invention, les additifs habituels présents dans ces types de formulation. On peut citer en particulier :
- des agents tensioactifs anioniques, tels que les sels hydrosolubles d'alkylsulfates, d'alkyléthersulfates, les alkyliséthionates et les alkyltaurates ou leurs sels, les alkylcarboxylates, les alkylsulfosuccinates ou les alkylsuccinamates, les alkylsarcosinates, les dérivés alkylés d'hydrolysats de protéines, les acylaspartates, les sophoroses lipides, ou encore les alkyl-D-galactosides uronates de formule 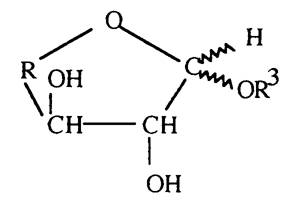 R³ étant un radical linéaire ou ramifié alkyle de 6 à 22 atomes de carbone et de préférence de 8 à 16 atomes de carbone, hydrocarboné ayant de 1 à 4 insaturations éthyléniques et de 6 à 22 atomes de carbone ou l'un de ces radicaux substitués par 1 à 3 substituants sur des atomes de carbone différents choisis parmi hydroxy, halogène et trifluorométhyle,
   R étant

   〉CH-CH (OH)-CO₂R⁴

   ou 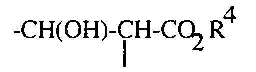 dont le carbone portant le groupe hydroxy n'est pas relié à l'atome d'oxygène endocyclique. R⁴ étant l'hydrogène, un atome de métal alcalin, de métal alcalino-terreux, un groupe ammonium quaternaire ;
- des agents tensioactifs zwitterioniques ou amphotères, comme les alkylbétaïnes, les alkylamidopropylbétaïnes, les dérivés d'imidazoline tels que les alkylamphoacétates, alkylamphodiacétates, alkylamphopropionates, alkylamphodipropionates, les alkylsultaines ou les alkylamidopropylhydroxysultaines
- des agents tensioactifs non-ioniques, comme les dérivés polyoxyéthylénés d'alcools aliphatiques ou arylaliphatiques en C8-C22, les alkylpolysaccharides présentant un groupement hydrophobe en C6-C30, de préférence en C10-C16 et un groupement polysaccharide, par exemple polyglycoside, comme groupement hydrophile ainsi que de 1 à 3 unités sucre, les dérivés alkylés d'aminosucres, comme les alkylglucamides produits par la réaction d'amidification d'un acide gras sur la N-méthylglucamine
- des amides dérivés d'acides gras utilisés généralement pour leur aptitude à améliorer le moussage des compositions ou à augmenter la viscosité desdites compositions
- des agents conditionneurs (améliorant la peignabilité, le coiffage, le toucher et le volume de la chevelure) cationiques tels que les polymères cationiques synthétiques du type MIRAPOL AD® ou PIRAPOL A550® commercialisés par RHONE-POULENC, des polymères cationiques naturels comme les dérivés cationiques du guar (JAGUAR C135®, JAGUAR C162® commercialisés par RHONE-POULENC) ou les dérivés cationiques de la cellulose (POLYMER JR400® commercialisé par UNION CARBIDE).
- des organopolysiloxanes utilisés tels quels ou en solution dans un de leurs solvants usuels (huiles silicones de basse masse, huiles paraffiniques très ramifiées, les esters gras par exemple du type palmitate d'isopropyle,...); comme agents conditionneurs ou de brillance.
- des polymères amphotères comme par exemple les copolymères de chlorure de diméthyldiallylammonium ou de diéthyldiallylammonium et d'acide acrylique.
- des agents antipelliculaires, comme les pyridinethiones, plus spécialement la zinc pyridinethione, les composés à base de sélénium comme le sulfure de sélénium ou l'OCTOPYROX®, commercialisé par HOECHST
- des agents anti-parasites (anti-poux), comme le LINDANE ou différentes pyréthrines utilisées à ce propos.

Ces compositions peuvent aussi contenir des agents modifiant l'aspect ou la viscosité des formulations comme les composés perlescents à base de stéarate de polyéthylène glycol ou des polymères améliorant la viscosité ou la stabilité comme les CARBOPOL® commercialisés par GOODRICH, les hydrocolloïdes et leurs dérivés comme le guar ou les guars modifiés, la caroube, la gomme xanthane, les dérivés de la cellulose (hydroxyéthylcellulose, carboxyméthylcellulose).
Les compositions classiques de shampooings contenant les tensioactifs non-ioniques de l'invention, peuvent être constituées de (pourcentages en poids)

| | |
|---|---|
| . pentosides d'alkyle de l'invention | 1-15% |
| . agent tensioactif anionique | 0-15% |
| . agent tensioactif amphotère | 0-10% |
| . alcanolamide d'acide gras | 0-5% |
| . agent épaississant | 0-5% |
| . agent conditionneur | 0-3% |
| . parfum | 0-2% |
| . conservateur | 0-2% |
| . agent antipelliculaire | 0-2% |
| . eau | complément à 100% |

Les tensioactifs non-ioniques de l'invention peuvent être utilisés également dans des formulations où il est nécessaire de maintenir en suspension dans l'eau des solides finement divisés, comme les formulations de matières actives agrochimiques (herbicides, insecticides, fongicides,...) connues sous le nom générique de "suspensions concentrées". Outre un tensioactif dispersant, on retrouve comme additifs dans une formulation de suspension concentrée, des additifs comme ceux décrits dans la brochure commerciale "Auxiliaries for agrochemical formulations" éditées par RHONE-POULENC GERONAZZO SpA.
On peut citer par exemple un tensioactif mouillant, pris parmi les dérivés alcoylés d'alcools arylaliphatiques, les dérivés aryl sulfonés comme l'isopropylnaphtalène sulfonate de sodium, commercialisé sous le nom SUPRAGIL WP® par RHONE-POULENC GERONAZZO, les dialkyl sulfosuccinates comme le di-éthyl-2-hexyl sulfosuccinate de sodium, des polymères dispersants, comme les acides polyacryliques et leurs sels, les copolymères anhydride (ou acide) maléique - diisobutylène et leurs sels comme le GEROPON T36® (RHONE-POULENC GERONAZZO), les méthylnaphtalène sulfonates de sodium condensés comme le SUPRAGIL MNS90® (RHONE-POULENC GERONAZZO), les polymères dispersants dérivés de la lignine comme les lignosulfonates de sodium ou de calcium ou d'autres tensioactifs dispersants comme les dérivés alcoxylés, éventuellement sulfatés ou phosphatés de tristyrylphénols. On peut trouver en outre dans ces formulations, des additifs antigels comme le propylèneglycol et des additifs épaississants, modifiant le comportement rhéologique de la suspension comme la gomme xanthane, les dérivés de la cellulose (carboxyméthylcellulose), la gomme guar ou ses dérivés, des argiles ou des argiles modifiées comme la bentionite et les bentones.
Parmi les matières actives pouvant être formulées ainsi, on trouve généralement celles dont le point de fusion est supérieur à 45°C préférentiellement supérieur à 60°C et dont la solubilité dans l'eau est inférieure à 10 g/l, préférentiellement inférieure à 1 g/l. Les matières actives phytosanitaires concernées sont les herbicides, les fongicides et insecticides, tels que ceux décrits dans THE PESTICIDE MANUAL (9° édition, C.R. WORKLING et R.J. HANCE, éditeurs, publié par The British Crop Protection Council) et répondant aux critères ci-dessus. Les exemples suivants illustrent l'invention.

### Exemple 1

On met en contact 326 g de son désamidonné séché à 92 % de matière sèche contenant par rapport à la matière sèche 0,2 % d'amidon, 11,5 % de protéines, 46 % de pentosanes et 700 g de solution d'acide chlorhydrique à 1,8 %. Le milieu réactionnel est homogénéisé dans un réacteur agité, puis porté à une température de 107 °C pendant 30 minutes. Après le choc thermique, le mélange est pressé sur une presse de laboratoire CARVER pour séparer le marc 1 du jus 1. On recueille un jus et un marc à respectivement 13,8 % et 50 % de matière sèche. Le jus est déminéralisé par échange d'ions sur une résine cationique forte (IRA 200) et une résine anionique faible (IRA 945). Le jus est ensuite concentré jusqu'à 72 % de matière sèche par évaporation de l'eau sous pression réduite ; sa composition est reportée dans le tableau suivant :

| | JUS 1 |
|---|---|
| Matière sèche (%) | 72,0 |
| L-Arabinose / Matière sèche (%) | 29,5 |
| D-Xylose / Matière sèche (%) | 54,8 |
| autres sucres / Matière sèche (%) | 11,2 |
| Pentoses/ Matière sèche (%) | 84,3 |

Le jus présente une pureté en sucres neutres de 95,5 %, les pentoses représentant 84,3 % de la matière sèche.

20 g de ce jus sont ensuite mis en suspension dans 25 g de *n*-butanol en présence de 0,26 g de catalyseur acide. Le milieu réactionnel est porté à 80 °C et l'eau est éliminée au cours de la réaction. On ajoute ensuite à la même température l'alcool gras 31 g (décanol : 85 %, dodécanol : 15 %) et le butanol est éliminé sous pression réduite. L'acidité du milieu est neutralisée par une solution d'hydrogénocarbonate de sodium saturée jusqu' à pH 7 à 8. Les alcools gras en excès sont éliminés par évaporation sous pression réduite (1 à 5 mb) à une température comprise entre 140 et 160 °C. Le résidu (21 g) se présente sous la forme d'une pâte qui contient moins de 2 % d'alcool gras résiduel. Celle-ci est mise en solution à 70 % dans l'eau et est décolorée en présence d'eau oxygénée

### Exemple 2

On met en contact 326 g de son désamidonné séché à 92 % de matière sèche contenant par rapport à la matière sèche 0,2 % d'amidon, 11,5 % de protéines, 46 % de pentosanes et 700 g de solution d'acide chlorhydrique à 1,8 %. Le milieu réactionnel est homogénéisé dans un réacteur agité, puis porté à une température de 107 °C pendant 30 minutes. Après le choc thermique, le mélange est pressé sur une presse de laboratoire CARVER pour séparer le marc 1 du jus 1. On recueille un jus et un marc à respectivement 13,8 % et 50 % de matière sèche. La composition du jus 1 est reportée dans le tableau suivant :

| | JUS 1 |
|---|---|
| Matière sèche (%) | 13,8 |
| L-Arabinose / Matière sèche (%) | 25,8 |
| D-Xylose / Matière sèche (%) | 44,0 |
| autres sucres / Matière sèche (%) | 12,3 |
| pentoses / Matière sèche (%) | 69,8 |

Le jus présente une pureté en pentoses de 82,1 %, les pentoses représentant 69,8 % de la matière sèche. Ce jus est ensuite concentré jusqu'à une matière sèche de 49 % par évaporation de l'eau sous pression réduite.

50 g de ce jus sont ensuite mis en suspension dans 96 g d'une coupe d'alcools gras (octanol : 50 %, décanol : 50 %) contenant 2,5 g de matière sèche de tensioactif de l'exemple 1. Le milieu réactionnel est porté à 80 °C sous pression réduite (50 mb) et après 5 heures de réaction, l'acidité de ce milieu est neutralisée par une solution d'hydrogénocarbonate de sodium saturée jusqu' à pH 7 à 8. Les alcools gras en excès sont éliminés par évaporation sous pression réduite (1 mb) à une température comprise entre 120 et 150 °C. Le résidu obtenu (32 g) contient moins de 1 % d'alcool gras résiduel.

### Exemple 3

Le procédé est réalisé selon le protocole décrit précédemment dans l'exemple 2 si ce n'est que l'on purifie les pentosides d' octyl-décyle par chromatographie sur colonne de gel de silice Merck 60H ; éluant : chlorure de méthylène puis chlorure de méthylène/méthanol (9/1, v/v). On recueille alors 15,3 g de pentosides qui se présentent sous forme d'une pâte contenant moins de 0,5 % d'alcool gras résiduel.

### Exemple 4

On met en contact 159 kg de son désamidonné à 36 % de matière sèche (contenant 0,3 kg d'amidon, 6,7 kg de protéines, 25 kg de pentosanes), 149 kg d'eau et 6,0 kg d'acide sulfurique à 95 %. Le milieu réactionnel est homogénéisé dans un réacteur agité, puis porté à une température de 140 °C par un jet de vapeur (44 kg) pendant 30 minutes. Après le choc thermique, le mélange est pressé sur une presse à vis hélicoïdales à section libre décroissante, de l'entrée à la sortie, dotées de tamis à fentes transversales perpendiculaires à l'axe de la presse. La largeur des fentes est de 0,5 à 0,25 mm. La pression de gavage des presses est de 0,1 à 0,2. 10⁵ Pa, et la pression maximale à laquelle est soumise le son dans la partie la plus étroite est de 5.10⁵ Pa. Le marc 1 (79 kg) est alors séparé du jus 1 (279 kg). Les compositions du jus 1 et du marc 1 sont reportées dans le tableau suivant :

| | Marc 1 | JUS 1 |
|---|---|---|
| Matière sèche (%) | 38,9 | 12,9 |
| Amidon / Matière sèche (%) | 0 | 0,8 |
| Protéines / Matière sèche (%) | 10 | 10 |
| Equivalents pentoses / Matière sèche (%) | 11,6 | 70 |

Le marc 1 est directement repris à la sortie de la presse par une pompe et dilué en ligne avec 236 l d'eau. Il repasse alors sur une seconde presse identique à la précédente où l'on récupère de nouveau deux fractions, le jus 2 ( 262 kg) et le marc 2 (54 kg). Leur composition est reportée dans le tableau suivant :

| | Marc 2 | JUS 2 |
|---|---|---|
| Matière sèche (%) | 39,1 | 3,7 |
| Amidon / Matière sèche (%) | 0 | 0 |
| Protéines / Matière sèche (%) | 11,9 | 6 |
| Equivalents pentoses / Matière sèche (%) | 4,8 | 25 |

Les fractions de jus 1 et 2 sont rassemblées chaulées en présence de 22 kg de lait de chaux à 20 % de matière sèche. Après centrifugation sur une centrifugeuse à assiettes auto-débourbeuse on obtient le jus clair 3 ( 534 kg) dépourvu d'insolubles et les crèmes (28,1 kg). Les bilans matières sont reportés dans le tableau suivant :

| | Crèmes | Jus clair 3 |
|---|---|---|
| Matière sèche (%) | 18,0 | 8,4 |
| Amidon / Matière sèche (%) | 2 | 0,5 |
| Protéines / Matière sèche (%) | 34 | 5,6 |
| Equivalents pentoses / Matière sèche (%) | 12 | 60,2 |

Le jus clair 3 est ensuite déminéralisé par échange d'ions sur une résine cationique forte (IR 200) et une résine anionique faible (IRA 94S). Le jus 4 obtenu (561 kg à 6 % de matière sèche) est ensuite concentré jusqu'à 70 % de matière sèche par évaporation de l'eau sous pression réduite ; sa composition est reportée dans le tableau suivant :

| | JUS 4 |
|---|---|
| Matière sèche (%) | 70,0 |
| Amidon / Matière sèche (%) | 0,6 |
| Protéines/ Matière sèche (%) | 1,5 |
| Pentoses/ Matière sèche (%) | 79,7 |

Ce jus est ensuite mis en suspension dans 65 kg de *n*-butanol en présence de 0,7 kg de catalyseur acide. Le milieu réactionnel est porté à 80 °C et l'eau est éliminée au cours de la réaction. On ajoute ensuite à la même température l'alcool gras 73,3 kg (décanol : 85 %, dodécanol : 15 %) et le butanol est éliminé sous pression réduite. L'acidité du milieu est neutralisée par une solution aqueuse de soude à 32 %. Les alcools gras en excès sont éliminés par évaporation sous pression réduite (1 à 5 mb) à une température comprise entre 140 et 160 °C. Le résidu (58,8 kg) se présente sous la forme d'une pâte qui contient moins de 1 % d'alcool gras résiduel. Celle-ci est mise en solution à 60 % dans l'eau et est décolorée en présence de 3,5 kg d'eau oxygénée à 50 %.

### Exemple 5

On met en contact 109kg de paille broyée à 92 % de matière sèche (contenant 33,5 kg de cellulose, 3,0 kg de protéines, 25 kg de pentosanes et 16,5 kg de lignine), 805 kg d'eau et 10,5 kg d'acide sulfurique à 95 %. Le milieu réactionnel est homogénéisé dans un réacteur agité, puis porté à une température de 140 °C par un jet de vapeur (91 kg) pendant 30 minutes. Après le choc thermique, le mélange est pressé sur une presse à vis hélicoïdales à section libre décroissante, de l'entrée à la sortie, dotées de tamis à fentes transversales perpendiculaires à l'axe de la presse. La largeur des fentes est de 0,5 à 0,25 mm. La pression de gavage des presses est de 0,1 à 0,2. 10⁵ Pa, et la pression maximale à laquelle est soumise la paille dans la partie la plus étroite est de 5.10⁵ Pa. Le marc 1 (183 kg) est alors séparé du jus 1 (832 kg). Les compositions du jus 1 et du marc 1 sont reportées dans le tableau suivant :

| | Marc 1 | JUS 1 |
|---|---|---|
| Matière sèche (%) | 41,5 | 4,3 |
| Cellulose / Matière sèche (%) | 43,1 | 2,0 |
| Protéines / Matière sèche (%) | 3,0 | 2,0 |
| Lignine / Matière sèche (%) | 23,7 | 1,1 |
| Equivalents pentoses / Matière sèche (%) | 5,3 | 62,5 |

Le marc 1 est directement repris à la sortie de la presse par une pompe et dilué en ligne avec 548 l d'eau. Il repasse alors sur une seconde presse identique à la précédente où l'on récupère de nouveau deux fractions, le jus 2 ( 621 kg) et le marc 2 (110 kg). Leur composition est reportée dans le tableau suivant :

| | Marc 2 | JUS 2 |
|---|---|---|
| Matière sèche (%) | 50 | 3,4 |
| Cellulose / Matière sèche (%) | 60,0 | 0 |
| Protéines / Matière sèche (%) | 1,3 | 7,6 |
| Lignine / Matière sèche (%) | 32,7 | 0 |
| Equivalents pentoses / Matière sèche (%) | 1,0 | 16,1 |

Les fractions de jus 1 et 2 sont rassemblées chaulées en présence de 65 kg de lait de chaux à 20 % de matière sèche. Après centrifugation sur une centrifugeuse à assiettes auto-débourbeuse on obtient le jus clair 3 ( 1352 kg) dépourvu d'insolubles et les crèmes (167,1 kg). Les bilans matières sont reportés dans le tableau suivant :

| | Crèmes | JUS 3 |
|---|---|---|
| Matière sèche (%) | 10 | 3,0 |
| Cellulose / Matière sèche (%) | 4,1 | 0 |
| Protéines / Matière sèche (%) | 1,8 | 5,0 |
| Lignine / Matière sèche (%) | 2,3 | 0 |
| Equivalents pentoses / Matière sèche (%) | 3,0 | 63,2 |

Le jus clair 3 est ensuite déminéralisé par échange d'ions sur une résine cationique forte (IR 200) et une résine anionique faible (IRA 94S). Le jus 4 obtenu (1420 kg à 2,2 % de matière sèche) est ensuite concentré jusqu'à 70 % de matière sèche par évaporation de l'eau sous pression réduite ; sa composition est reportée dans le tableau suivant :

| | JUS 4 |
|---|---|
| Matière sèche (%) | 70,0 |
| Cellulose/ Matière sèche (%) | 0,0 |
| Protéines/ Matière sèche (%) | 1,5 |
| Pentoses/ Matière sèche (%) | 80,6 |

Ce jus est ensuite mis en suspension dans 60,6 kg de *n*-butanol en présence de 0,6 kg de catalyseur acide. Le milieu réactionnel est porté à 80 °C et l'eau est éliminée au cours de la réaction. On ajoute ensuite à la même température l'alcool gras 68,2 kg (décanol : 85 %, dodécanol : 15 %) et le butanol est éliminé sous pression réduite. L'acidité du milieu est neutralisée par une solution aqueuse de soude à 32 %. Les alcools gras en excès sont éliminés par évaporation sous pression réduite (1 à 5 mb) à une température comprise entre 140 et 160 °C. Le résidu (55,5 kg) se présente sous la forme d'une pâte qui contient moins de 1 % d'alcool gras résiduel. Celle-ci est mise en solution à 70 % dans l'eau et est décolorée en présence de 3,3 kg d'eau oxygénée à 50 %.

### Exemple 6

On met en contact 217 kg de paille broyée à 92 % de matière sèche, 185 kg d'eau, 455 kg de jus 2 de l'exemple 5, 240 kg de jus 1 de l'exemple 5 et 15 kg d'acide sulfurique à 95 %. Le milieu réactionnel est homogénéisé dans un réacteur agité, puis porté à une température de 140 °C pendant 30 minutes. Après le choc thermique, le mélange est pressé sur une presse bivis STORD MS 35. Le marc 1 est alors séparé du jus 1 (480 kg). La composition du jus 1 est reportée dans le tableau suivant :

| | JUS 1 |
|---|---|
| Matière sèche (%) | 15 |
| Pentoses / Matière sèche (%) | 55 |

Le marc 1 est directement repris à la sortie de la presse par une pompe et dilué en ligne avec 450 l d'eau. Il repasse alors sur une seconde presse identique à la précédente où l'on récupère de nouveau deux fractions, le jus 2 ( 455 kg) et le marc 2. Le jus 2 est renvoyé pour l'hydrolyse d'un nouveau batch de paille.

Le jus 1 est chaulé avec 50 ml de lait de chaux à 180 g/l par litre de jus. Après centrifugation sur une centrifugeuse à assiettes auto-débourbeuse on obtient le jus clair 3 dépourvu d'insolubles et les crèmes.

Le jus clair 3 est ensuite déminéralisé par échange d'ions sur une résine cationique forte (IR 200) et une résine anionique faible (IRA 945). Le jus 4 obtenu est ensuite concentré jusqu'à 75 % de matière sèche par évaporation de l'eau sous pression réduite ; sa composition est reportée dans le tableau suivant :

| | JUS 4 |
|---|---|
| Matière sèche (%) | 75,0 |
| Pentoses (kg) | 45 |
| Protéines/ Matière sèche (%) | 1,5 |
| Pentoses/ Matière sèche (%) | 90 |

Ce jus est ensuite mis en suspension dans 83 kg de *n*-butanol en présence de 0,5 kg de catalyseur acide. Le milieu réactionnel est porté à 80 °C et l'eau est éliminée au cours de la réaction. On ajoute ensuite à la même température l'alcool gras 93 kg (octanol : 50 %, décanol : 50 %) et le butanol est éliminé sous pression réduite. L'acidité du milieu est neutralisée par une solution aqueuse de soude à 32 %. Les alcools gras en excès sont éliminés par évaporation sous pression réduite (1 à 5 mb) à une température comprise entre 120 et 140 °C. Le résidu (83,6 kg) se présente sous la forme d'une pâte qui contient moins de 1 % d'alcool gras résiduel. Celle-ci est mise en solution à 70 % dans l'eau et est décolorée en présence de 5 kg d'eau oxygénée à 50 %.

### Exemple 7

On prépare une suspension de gros son à 20 % de matière sèche (100 g de matière sèche son pour 500 g de suspension) dans une solution d'acide sulfurique (10 % H₂SO₄ / MS son). Le milieu réactionnel est homogénéisé dans un réacteur agité, puis porté à une température de 130 °C pendant 30 minutes. Après le choc thermique, le mélange est presse sur une presse MARREL pour séparer le marc 1 du jus 1. On recueille un jus et un marc à respectivement 17,37 % et 53,0 % de matière sèche. La composition du jus 1 est reportée dans le tableau suivant :

| | JUS 1 |
|---|---|
| Matière sèche (%) | 17,4 |
| Pentoses / Matière sèche (%) | 49 |

Le jus 1 est chaulé (16,4 % (v/v)) avec du lait de chaux à 30 % de matière sèche. Après centrifugation sur une centrifugeuse à assiettes auto-débourbeuse on obtient le jus clair 2 dépourvu d'insolubles et les crèmes.

Le jus clair 2 est ensuite déminéralisé par échange d'ions sur une résine cationique forte (IR 200) et une résine anionique faible (IRA 94S). Le jus 3 obtenu est ensuite concentré jusqu'à 75 % de matière sèche par évaporation de l'eau sous pression réduite ; sa composition est reportée dans le tableau suivant :

| | JUS 3 |
|---|---|
| Matière sèche (%) | 75,0 |
| Protéines/ Matière sèche (%) | 1,5 |
| Pentoses/ Matière sèche (%) | 90 |

100 g de ce jus sont ensuite mis en suspension dans 114 g de *n*-butanol en présence de 1,3 g de catalyseur acide. Le milieu réactionnel est porté à 100 °C et l'eau est éliminée au cours de la réaction. On ajoute ensuite à la même température l'alcool gras 156 g (dodécanol) et le butanol est éliminé sous pression réduite. L'acidité du milieu est neutralisée par une solution aqueuse de soude à 32 %. Les alcools gras en excès sont éliminés par évaporation sous pression réduite (1 à 5 mb) à une température comprise entre 160 et 170 °C. Le résidu (104 g) se présente sous la forme d'une pâte qui contient moins de 1 % d'alcool gras résiduel. Celle-ci est mise en solution à 50 % dans l'eau et est décolorée en présence de 6 g d'eau oxygénée à 50 %.

### Exemple 8

On met en contact 4762 g de fibres de blé à 21 % de matière sèche, 6138 g d'eau et 100 g d'acide sulfurique à 95 %. Le milieu réactionnel est homogénéisé dans un réacteur agité, puis porté à une température de 140 °C, par un jet de vapeur (1620 g), pendant 30 minutes. Après le choc thermique, on obtient un jus 1 (12620 g) qui présente une matière sèche de 8,7 %.

Ce jus 1 est chaulé avec 505 g de lait de chaux à 18 % de matière sèche. Après centrifugation sur une centrifugeuse à assiettes auto-débourbeuse on obtient le jus clair 2 dépourvu d'insolubles et les crèmes.

Le jus clair 2 est ensuite déminéralisé par échange d'ions sur une résine cationique forte (IR 200) et une résine anionique faible (IRA 94S). Le jus 3 obtenu est ensuite concentré jusqu'à 70 % de matière sèche par évaporation de l'eau sous pression réduite ; sa composition est reportée dans le tableau suivant :

| | JUS 3 |
|---|---|
| Matière sèche (%) | 70,0 |
| Masse (g) | 1139 |
| Pentoses (g) | 702 |
| Protéines/ Matière sèche (%) | 1,5 |
| Pentoses/ Matière sèche (%) | 88 |

Ce jus est ensuite mis en suspension dans 1215 g de *n*-butanol en présence de 11,9 g de catalyseur acide. Le milieu réactionnel est porté à 105 °C et l'eau est éliminée au cours de la réaction. On ajoute ensuite à la même température l'alcool gras 1408 g (décanol) et le butanol est éliminé sous pression réduite. L'acidité du milieu est neutralisée par une solution aqueuse de soude à 32 %. Les alcools gras en excès sont éliminés par évaporation sous pression réduite (1 à 5 mb) à une température comprise entre 140 et 160 °C. Le résidu (925 g) se présente sous la forme d'une pâte qui contient moins de 1 % d'alcool gras résiduel. Celle-ci est mise en solution à 70 % dans l'eau et est décolorée en présence de 60 g d'eau oxygénée à 50 %.

### Exemple 9

Le jus 3 de l'exemple 8 est ensuite mis en suspension dans 1215 g de *n*-butanol en présence de 11,9 g de catalyseur acide. Le milieu réactionnel est porté à 105 °C et l'eau est éliminée au cours de la réaction. On ajoute ensuite à la même température l'alcool gras 1283 g (octanol/décanol = 1/1) et le butanol est éliminé sous pression réduite. L'acidité du milieu est neutralisée par une solution aqueuse de soude à 32 %. Les alcools gras en excès sont éliminés par évaporation sous pression réduite (1 à 5 mb) à une température comprise entre 120 et 160 °C. Le résidu (909 g) se présente sous la forme d'une pâte qui contient moins de 1 % d'alcool gras résiduel. Celle-ci est mise en solution à 70 % dans l'eau et est décolorée en présence de 60 g d'eau oxygénée à 50 %.

### Exemple 10

Le procédé est réalisé selon le protocole décrit précédemment dans l'exemple 1 si ce n'est que l'on utilise un mélange d'hexadécanol et d'octadécanol comme alcool gras et qu'on purifie les pentosides d' alkyle par chromatographie sur colonne de gel de silice Merck 60H ; éluant : chlorure de méthylène puis chlorure de méthylène/méthanol (9/1, v/v). On recueille alors 23 g de pentosides contenant moins de 0,5 % d'alcool gras résiduel.

### Exemple 11

Le procédé est réalisé selon le protocole décrit précédemment dans l'exemple 1 si ce n'est que l'on utilise un mélange d'hexadécanol et d'octadécanol comme alcool gras et que l'on n'élimine pas les alcools gras en fin de synthèse. On recueille alors 59 g de pentosides contenant 51 % d'alcool gras résiduel.

### Exemple 12

### Réactivités relatives du D-xylose, du L-arabinose et du D-glucose

Le sucre est mis en suspension dans 4 équivalents d'octanol en présence de 3 % en masse par rapport au sucre d'acide sulfurique. L'eau de la réaction est éliminée sous pression réduite 90 mb au fur et à mesure de sa formation. L'avancement de la réaction est suivi par CPG après silylation du milieu réactionnel. Les résultats sont énoncés dans la tableau suivant.

| **Sucres** | **T = 90°C** | |
|---|---|---|
| | **Temps de réaction** | **remarque** |
| D-xylose | 60 min | milieu limpide |
| L-arabinose | 90 min | milieu limpide |
| D-glucose | 19h | milieu marron + solide |
| L-arabinose/D-glucose 80/20 | 3 h | milieu limpide et marron |
| Sirop de son de l'exemple 4 (jus 4) | 3h | milieu limpide et peu coloré |

### Glycosidations comparatives des pentoses et des hexoses constituant les sirops de son

Dans les conditions de réaction définies précédemment, il est clair que les pentoses réagissent plus vite que le glucose pendant la glycosylation. Pour engager l'ensemble du glucose en quelques heures il serait alors nécessaire d'élever la température de réaction au dessus de 90°C. Nous avons d'autre part montré que si la réaction est réalisée à partir de L-arabinose et de D-glucose dans les proportions 80/20, le glucose s'engageait plus vite.

### Exemple 13

| Pouvoirs moussants de pentosides d'alkyle selon l'invention R¹ = C8, C10, C12 | | | |
|---|---|---|---|
| Tensioactif | Alcool gras | Concentration (%) | Volume de mousse (ml) |
| exemple 1 | C10-C12 | 0,1 | 460 |
| | | 1,0 | 540 |
| exemple 2 | C8-C10 | 0,1 | 355 |
| | | 1,0 | - |
| exemple 3 | C8-C10 | 0,1 | 360 |
| | | 1,0 | 420 |
| exemple 4 | C10-C12 | 0,1 | 500 |
| | | 1,0 | 530 |
| exemple 5 | C10-C12 | 0,1 | 490 |
| | | 1,0 | 510 |
| exemple 6 | C8-C10 | 0,1 | 495 |
| | | 1,0 | 520 |
| exemple 7 | C12 | 0,1 | 390 |
| | | 1,0 | - |
| exemple 8 | C10 | 0,1 | 505 |
| | | 1,0 | - |
| exemple 9 | C8-C10 | 0,1 | 460 |
| | | 1,0 | 560 |

### Exemple 14

| Mesures comparatives de pouvoirs moussants de pentosides d'alkyle selon l'invention R¹ = C8-C10,C10, C10-C12, C12 | | | |
|---|---|---|---|
| Tensioactif | Chaîne alkyle | Concentration (%) | Volume de mousse (ml) |
| exemple 4 | C10-C12 | 0,1 | 500 |
| | | 1,0 | 530 |
| exemple 5 | C10-C12 | 0,1 | 490 |
| | | 1,0 | 510 |
| exemple 6 | C8-C10 | 0,1 | 495 |
| | | 1,0 | 520 |
| exemple 8 | C10 | 0,1 | 505 |
| | | 1,0 | - |
| exemple 9 | C8-C10 | 0,1 | 460 |
| | | 1,0 | 560 |
| Dodécylbétäine | C12 | 0,1 | 400 |
| | | 1,0 | - |
| LES | C12 | 0,1 | 420 |
| | | 1,0 | 460 |
| ORAMIX CG 110 | C8-C10 | 0,1 | 460 |
| | | 1,0 | - |
| PLANTAREN 2000 | C8-C16 | 0,1 | 465 |
| | | 1,0 | - |

### Exemple 15

| Stabilité de la mousse de pentosides d'alkyle selon l'invention R¹ = C8-C10, C10, C12, C10-C12 | | | | | |
|---|---|---|---|---|---|
| | Tensioactif à 0,1 % | | | | |
| Temps (min) | Exemple 4 | Exemple 5 | Exemple 6 | Exemple 8 | Exemple 9 |
| 0 | 500 | 490 | 495 | 505 | 460 |
| 1 | 500 | 485 | 490 | 500 | 455 |
| 2 | 490 | 480 | 485 | 495 | 450 |
| 5 | 480 | 470 | 470 | 475 | 425 |
| 10 | 410 | 450 | 430 | 440 | 395 |
| 15 | 370 | 415 | 400 | 400 | 380 |
| 20 | 350 | 410 | 370 | 375 | 365 |

### Exemple 16

| Mesures comparatives de la stabilité de la mousse de pentosides d'alkyle selon l'invention R¹ = C8-C10,C10, C12, C10-C12 | | | | | | |
|---|---|---|---|---|---|---|
| | Tensioactif à 0,1 % | | | | | |
| Temps (min) | Exemple 4 | Exemple 8 | Dodécylbétaïne | LES | ORAMIX CG 110 | PLANTAREN 2000 |
| 0 | 500 | 505 | 400 | 460 | 460 | 465 |
| 1 | 500 | 500 | 390 | 430 | 455 | 460 |
| 2 | 490 | 495 | 390 | 420 | 450 | 460 |
| 5 | 480 | 475 | 360 | 370 | 440 | 450 |
| 10 | 410 | 440 | 330 | 300 | 430 | 430 |
| 15 | 370 | 400 | 310 | 80 | 415 | 420 |
| 20 | 350 | 375 | 290 | 0 | 410 | 415 |

### Exemple 17

| Mesures comparatives de tensions superficielles à une concentration supérieure à la CMC de pentosides d'alkyle selon l'invention R¹ = C8-C10, C10-C12 | |
|---|---|
| Tensioactif | γ_{CMC} (mN/m) |
| Glycosides de l'exemple 1 | 26,3 |
| Glycosides de l'exemple 2 | 26,6 |
| Glycosides de l'exemple 3 | 25,7 |
| Glycosides de l'exemple 4 | 26,2 |
| Glycosides de l'exemple 5 | 26,4 |
| Glycosides de l'exemple 6 | 26,7 |
| Glycosides de l'exemple 7 | 26,5 |
| Glycosides de l'exemple 8 | 26,5 |
| Glycosides de l'exemple 9 | 27,5 |
| LES | 35 |
| SDBS | 30 |
| ORAMIX NS 10 | 27,3 |
| PLANTAREN 2000 | 29,7 |

### Exemple 18

### Exemples de préparations moussantes

| Shampooings | |
|---|---|
| - Sclerotium gum | 1,15g |
| - Tensioactif de l'exemple 5 | 10g |
| - Colorants, parfums | qs |
| - Diéthanolamide d'acide gras de soja | 3g |
| - Eau | qsp 100g |

| Gel douche | |
|---|---|
| - Tensioactif de l'exemple 6 | 10g |
| - Diéthanolamide d'acide gras de soja | 7g |
| - Colorants, parfums | qs |
| - Eau | qsp 100g |

### Exemples N°19 à 21

| FORMULES DETERGENTES EN POUDRE | | | |
|---|---|---|---|
| | **19** | **20** | **21** |
| Lauryl benzène sulfonate | 2 | 0 | 5 |
| Décyl D-galactoside uronate de sodium | 10 | 20 | 10 |
| -oléfine sulfonate | 0 | 10 | 0 |
| Dodécyl D-galactoside uronate de sodium | 0 | 0 | 10 |
| Octyl D-galactoside uronate d'octyle | 4 | 4 | 0 |
| Décyl D-galactoside uronate décyle | 0 | 4 | 0 |
| Dodécyl D-galactoside uronate de dodécyle | 0 | 0 | 2 |
| Pentosides de l'exemple 1 | 2 | 2 | 10 |
| Huile silicone | 2 | 2 | 0,2 |
| Zéolithe 4 A | 25 | 20 | 25 |
| Nitrilotriacétate de sodium | 8 | 15 | 10 |
| Carbonate de sodium | 8 | 15 | 10 |
| Citrate de sodium | 1 | 0 | 0 |
| Argile | 0 | 4 | 3 |
| Perborate de sodium | 20 | 4 | 3 |
| Tétraacétyl éthylène diamine | 1 | 0 | 0 |
| Tétraacétate éthylène diamine | 0,5 | 0 | 0 |
| Gel de sodium de carboxyméthyl cellulose | 1 | 2 | 0,3 |
| Enzymes | 0,5 | 0,4 | 1 |
| Azurants optiques | 0,2 | 0,5 | 0,1 |
| Silicate de sodium | 4 | 10 | 10 |
| Parfums | qs | qs | qs |
| Eau | balance | balance | balance |

### Exemples N°22 à 24

| FORMULES DETERGENTES LIQUIDES | | | |
|---|---|---|---|
| | **22** | **23** | **24** |
| Alkyl benzène sulfonate | 5 | 0 | 5 |
| Savons | 10 | 0 | 15 |
| Ether sulfate d'alcools gras | 0 | 2 | 5 |
| Dodécyl D-galactoside uronate de sodium | 0 | 20 | 5 |
| Composés de l'exemple 2 | 10 | 3 | 10 |
| Ether d'alkyl poly (ethylène glycol) | 10 | 0 | 10 |
| Décyl D-galactoside uronate de sodium | 5 | 10 | 0 |
| Chlorure de diméthylammonium | 0 | 0 | 4 |
| Alkanolamide d'acide gras | 1 | 0 | 0 |
| Protéases | 0,5 | 0,5 | 0 |
| Citrate de sodium | 2 | 4 | 5 |
| Zéolithes | 0 | 20 | 0 |
| Silicate de sodium | 1 | 0 | 0 |
| Ethanol/propylène glycol | 10 | 8 | 13 |
| Polycarboxylates | 0 | 2 | 5 |
| Azurants optiques | qs | qs | qs |
| Stabilisants (mono éthanolamide) | 3 | 0 | 3 |
| Parfums | qs | qs | qs |
| colorants | qs | qs | qs |
| Eau | 12,5 | 30 | 28 |

### Exemples N°25 à 28

| FORMULATIONS DE LIQUIDES VAISSELLE | | | | |
|---|---|---|---|---|
| | **25** | **26** | **27** | **28** |
| Décyl D-galactoside uronate de sodium | 0 | 0 | 0 | 10 |
| Dodécyl D-galactoside uronate de sodium | 15 | 20 | 0 | 10 |
| Composés de l'exemple 3 | 5 | 2 | 15 | 5 |
| Mono éthanolamide d'acide gras éthoxylé | 5 | 5 | 5 | 0 |
| Lauryl diéthanolamide | 0 | 0 | 3 | 10 |
| Lauryl sulfate de sodium polyoxyéthylène | 0 | 0 | 10 | 0 |
| Chlorure de sodium | 3 | 3 | 0 | 0 |
| Acrylate de sodium | 0,2 | 0 | 0 | 0 |
| EDTA | 0,3 | 0 | 0 | 0 |
| Ethanol | 0 | 0 | 0 | 3 |
| Propylène glycol | 0 | 0 | 0,2 | 0 |
| Oxyde d'amide | 0 | 0 | 1 | 0 |
| Parfums | qs | qs | qs | qs |
| Colorants | qs | qs | qs | 13 |
| Conservateurs | 0,5 | 0,5 | qs | qs |
| Eau | qsp 100 | qsp 100 | qsp 100 | qsp 100 |

### Exemples N° 29 à 32

| FORMULATIONS DE SAVONS LIQUIDES DOUX | | | | |
|---|---|---|---|---|
| | **29** | **30** | **31** | **32** |
| Composé de l'exemple 4 | 10 | 15 | 10 | 5 |
| Dodécyl D-galactoside de sodium | 0 | 0 | 5 | 10 |
| Dodécyl D-galactoside uronate de dodécyle | 0 | 2 | 0 | 0 |
| Tétradécyl D-galactoside uronate de tétradécyle | 0 | 0 | 0 | 3 |
| Lauryl sulfate de sodium | 0 | 5 | 0 | 0 |
| Cocoyl isothionate de sodium | 5 | 0 | 0 | 0 |
| Sel de sodium d'alkyl peptide | 0 | 0 | 0 | 3 |
| Cocoamidopropyl bétaïne | 5 | 0 | 5 | 0 |
| Hydrolysat protéine de blé | 0 | 0 | 2 | 0 |
| Propylène glycol | 0 | 0 | 0 | 4 |
| Chlorure de sodium | 3 | 2 | 2 | 0 |
| E.D.T.A | 0,3 | 0,3 | 0,3 | 0,3 |
| Alcool cétylique | 0 | 0 | 0 | 3 |
| Huile minérale lourde | 0 | 0 | 0 | 15 |
| Cocoamidodiéthanolamide | 5 | 0 | 5 | 0 |
| Hydroxyméthyl cellulose | 0 | 0 | 0 | 0,5 |
| Conservateurs | qs | qs | qs | qs |
| Désinfectants | 0 | 0 | 0 | 0,2 |
| Parfums | qs | qs | qs | qs |
| Eau | qsp 100 | qsp 100 | qsp 100 | qsp 100 |

### Exemples N° 33 à 36

| FORMULATIONS DE SHAMPOOINGS | | | | |
|---|---|---|---|---|
| | **33** | **34** | **35** | **36** |
| Dodécyl D-galactoside uronate de sodium | 0 | 0 | 5 | 0 |
| Tétradécyl D-galactoside uronate de sodium | 10 | 0 | 5 | 5 |
| Lauryl éther sulfosuccinate de disodium | 0 | 5 | 0 | 0 |
| Cocoamidopropyl bétaïne à 30 % | 5 | 10 | 0 | 5 |
| Diéthanolamide d'acide gras de coprah | 0 | 0 | 0 | 5 |
| Composés de l'exemple 2 | 5 | 10 | 10 | 10 |
| Hydrolysat de protéines de blé | 0 | 0 | 0 | 2 |
| Octadécyl polyéthylène glycol | 10 | 0 | 0 | 0 |
| Triéthylène glycol | 1 | 2 | 0 | 0 |
| Chlorure de sodium | 0 | 1,5 | 1,5 | 2 |
| Conservateurs | 0,5 | 0,4 | 0,4 | 0,4 |
| E.D.T.A | 0,3 | 0,3 | 0,3 | 0,3 |
| Parfums | qs | qs | qs | qs |
| Eau | qsp 100 | qsp 100 | qsp 100 | qsp 100 |

### Exemples N° 37 à 38

| FORMULATIONS DE BAINS MOUSSANTS | | |
|---|---|---|
| | **37** | **38** |
| Dodécyl D-galactoside uronate de sodium | 5 | 10 |
| Composés de l'exemple 3 | 15 | 10 |
| Cocoyl monoéthanolamide | 5 | 5 |
| Lauryl amido propyl bétaïne | 2 | 0 |
| Lauryl amido diméthyl bétaïne | 0 | 2 |
| Triéthyléne glycol | 2 | 0 |
| Huile d'amande douce | 6 | 5 |
| Laurate de sorbitan éthoxylé | 2 | 2 |
| Dioléate de propylène glycol éthoxylé | 0 | 2 |
| Lauryl myristyl à 30 E | 2 | 0 |
| E.D.T.A | 0,3 | 0,3 |
| Chlorure de sodium | 2 | qs |
| Acrylate d'oléyle | 0 | 2 |
| Conservateurs | 0,5 | qs |
| Hexadécanol | 1 | 1 |
| Parfums | qs | qs |
| Eau | qsp 100 | qsp 100 |

### Exemples N° 39 et 40

| FORMULATIONS DE GEL DOUCHE | | |
|---|---|---|
| | **39** | **40** |
| Décyl D-galactoside uronate de sodium | 5 | 0 |
| Tétradécyl D-galactoside uronate de sodium | 0 | 5 |
| Hexadéxyl D-galactoside uronate de sodium | 0 | 1 |
| Composés de l'exemple 5 | 10 | 10 |
| Lauryl amidopropyl bétaïne | 2 | 0 |
| Gel acrylique | 0 | 0,2 |
| E.D.T.A | 0,3 | 0,3 |
| Chlorure de sodium | 3 | 0 |
| Agents nacrants | 0 | 5 |
| Parfums | 0,2 | 0 |
| Conservateurs | 0,5 | 0,5 |
| Eau | qsp 100 | qsp 100 |

### Exemples N° 41 et 46

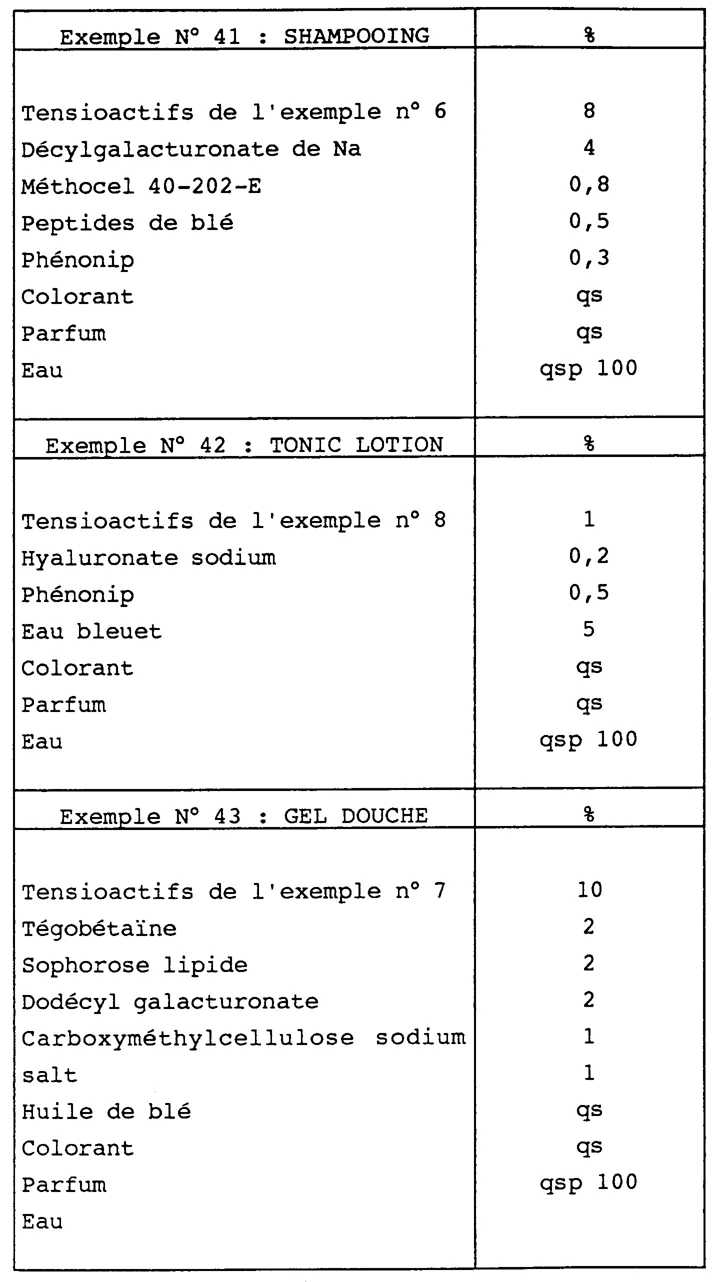

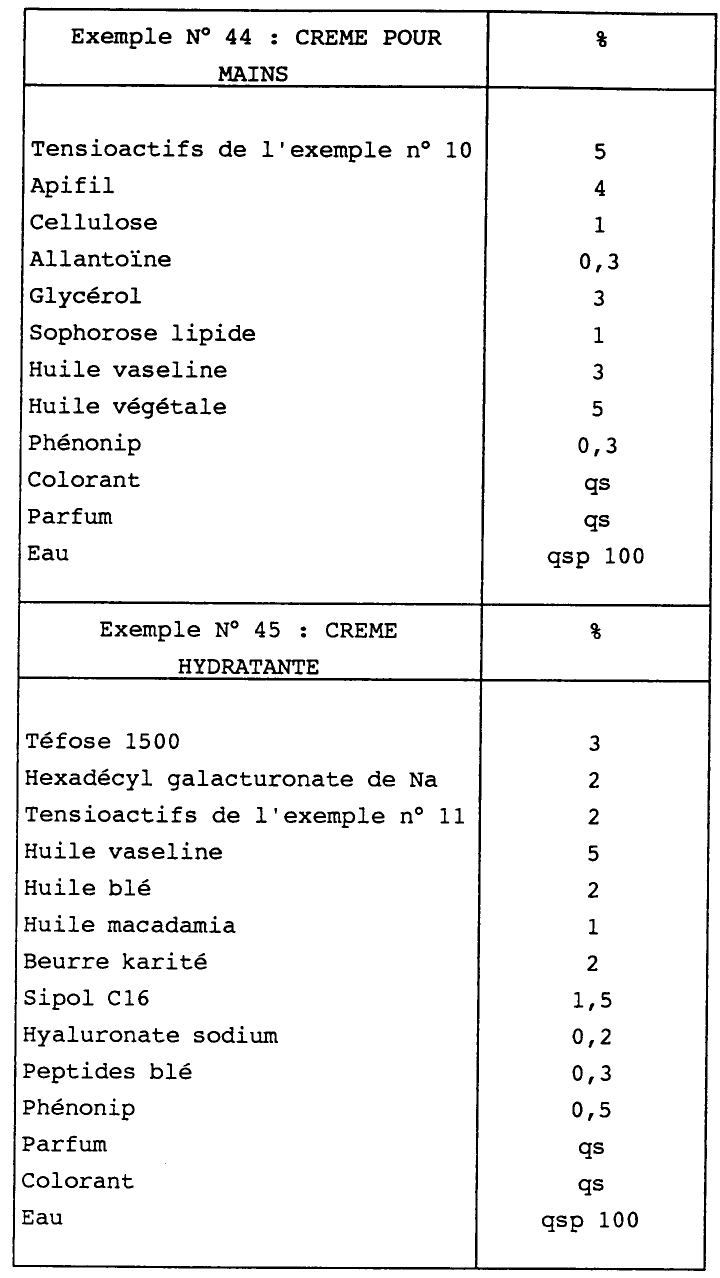

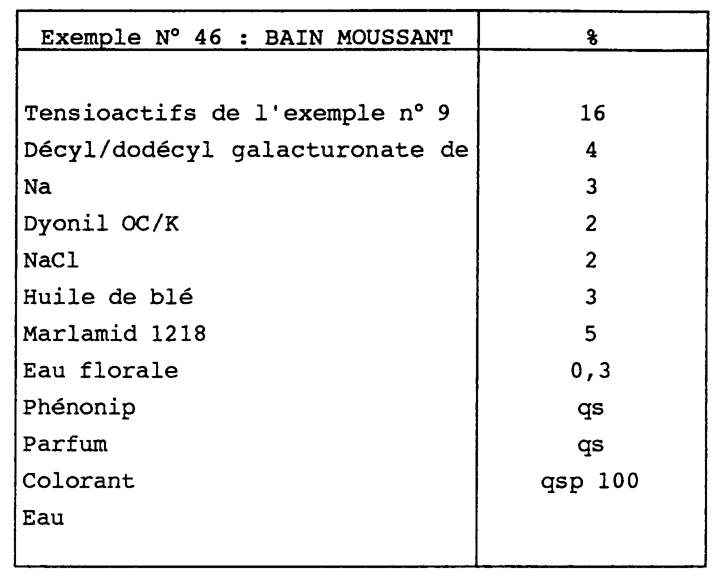

## Revendications

1. L'utilisation du son de blé, de la fibre de blé ou de la paille de blé comme matière première pour la préparation d'agents tensioactifs.

2. Procédé de préparation d'agent tensioactif, caractérisé en ce qu'il consiste à mettre du son de blé, de la fibre de blé ou de la paille de blé en contact avec une solution aqueuse acide entre 20 et 150°C pendant au moins 5 secondes pour obtenir un sirop de pentoses, à débarraser le sirop de pentoses d'un marc solide et à mettre le sirop de pentoses exempt de marc en contact avec un alcool ayant de 6 à 22 atomes de carbone à une température comprise entre 20 et 150°C jusqu'à obtention d'une solution de pentosides tensioactifs et à séparer le mélange des pentosides tensioactifs de cette solution.

3. Procédé suivant la revendication 2, caractérisé en ce qu'il consiste à effectuer la mise en contact du son de blé, de la fibre de blé ou de la paille de blé avec une solution acide aqueuse pendant 5 à 90 minutes.

4. Procédé suivant la revendication 2 ou 3, caractérisé en qu'il consiste à mettre le sirop de pentoses en contact avec un alcool à une température comprise entre 30 et 90°C.

5. Procédé suivant l'une des revendications 2 à 4, caractérisé en ce qu'il consiste, avant la mise en contact avec l'alcool, à déminéraliser le sirop de pentoses exempt de marc par chromatographie, par électrodialyse ou par passage sur au moins une résine échangeuse d'ions et, de préférence, par passage successivement sur une résine échangeuse de cations, puis sur une résine échangeuse d'anions, tout en laissant, de préférence au moins 0,02 équivalent H⁺ par mole de pentoses.

6. L'utilisation du mélange de pentosides pouvant être obtenu par le procédé de la revendication 2 comme agent tensioactif, notamment en détergence et en cosmétologie.

7. Composition détergente comprenant de 0,1 à 60% et, de préférence, de 10 à 30% en poids d'une base détergente et de 99,9 à 40% et, de préférence, de 90 à 70% en poids d'adjuvants, caractérisée en ce que la base détergente comprend de 1 à 100% en poids et, de préférence, de 50 à 100% en poids du mélange de pentosides pouvant être obtenu par le procédé de la revendication 2.

8. Composition cosmétique comprenant de 0,1 à 50% et, de préférence, de 5 à 35 % en poids de substance tensioactive et de 99,9 à 50% et, de préférence, de 95 à 65% en poids d'adjuvant ou de principe actif, caractérisée en ce que de 0,5 à 100% en poids des substances tensioactives sont constituées du mélange de pentosides pouvant être obtenu par le procédé de la revendication 2.

## Claims

1. Use of wheat bran, wheat fibre or wheat straw as raw material for the preparation of surface active agents.

2. Process for preparing a surface active agent, characterized in that wheat bran, wheat fibre or wheat straw is brought into contact with an aqueous acid solution at a temperature of between 20° and 150°C for at least 5 seconds in order to obtain a pentose syrup, the solid pulp is removed from the pentose syrup and the pentose syrup from which the pulp has been removed is brought into contact with an alcohol having from 6 to 22 carbon atoms at a temperature of between 20° and 150°C until a solution of surface active pentosides has been obtained and the mixture of surface active pentosides are removed from this solution.

3. Process as claimed in claim 2, in which the wheat bran, wheat fibre or wheat straw is brought into contact with an aqueous acid solution for 5-90 minutes.

4. Process as claimed in claim 2 or 3, in which the pentose syrup is brought into contact with an alcohol at a temperature of between 30° and 90°C.

5. Process as claimed in one of claims 2 to 4, in which before being brought into contact with the alcohol, the pentose syrup from which the pulp has been removed is demineralized by chromatography, electrodialysis, or by passing it through at least one ion exchange resin and preferably successively by passing it through a cation exchange resin, then an anion exchange resin, whilst leaving, in preference, at least 0.02 H⁺ equivalent per mole of pentoses.

6. Use of the mixture of pentosides obtainable by the process of claim 2 as a surface active agent, in particular in the field of detergent and cosmetics.

7. Detergent composition comprising from 0.1% to 60% by weight, and preferably 10 to 30% by weight, of a detergent base and from 99.9% to 40% by weight, and preferably from 90 to 70% by weight, of admixtures, characterized in that the detergent base comprises from 1% to 100% by weight, and preferably from 50% to 100% by weight, of the mixture of pentosides obtainable by the process in claim 2.

8. Cosmetic composition comprising from 0.1% to 50% by weight, and preferably from 5 to 35 % by weight, of surface active substances and from 99.0% to 50% by weight, and preferably from 95% to 65% by weight, of an admixture or active ingredient, characterized in that from 0.5% to 100% by weight of the surface active substances are formed from the mixture of pentosides obtainable by the process of claim 2.

## Patentansprüche

1. Verwendung von Weizenkleie, Weizenfasern oder Weizenstroh als Ausgangsmaterial für die Herstellung von Tensiden.

2. Verfahren zur Herstellung eines Tensids, dadurch gekennzeichnet, daß es aufweist:
Kontaktieren von Weizenkleie, Weizenfasern oder Weizenstroh mit einer wässrigen sauren Lösung zwischen 20 und 150°C während mindestens als 5 Sekunden, um einen Pentosen-Sirup zu erhalten,
Entfernen der festen Schlempe vom Pentosen-Sirup und Kontaktieren des Pentosen-Sirups ohne Schlempe mit einem Alkohol, der 6 bis 20 Kohlenstoffatome aufweist bei einer Temperatur zwischen 20 und 150 °C, bis eine Lösung oberfächenaktiver Pentoside erhalten wird und
Abtrennen der oberflächenaktiven Pentoside aus der Lösung.

3. Verfahren nach Anspruch 2, dadurch gekennzeichnet, daß das Kontaktieren von Weizenkleie, Weizenfasern oder Weizenstroh mit einer wässrigen sauren Lösung während 5 bis 90 Sekunden durchgeführt wird.

4. Verfahren nach Anspruch 2 oder 3, dadurch gekennzeichnet, daß das Kontaktieren des Pentose-Sirups mit einem Alkohol bei einer Temperatur zwischen 30 und 90°C durchgeführt wird.

5. Verfahren nach einem der Ansprüche 2 bis 4, dadurch gekennzeichnet, daß vor dem Kontaktieren mit Alkohol der Pentosen-Sirup ohne Schlempe durch Chromatographie, durch Elektrodialyse oder Passieren mindestens eines Ionenaustauscherharzes und bevorzugt aufeinanderfolgendes Passieren eines Kationenaustauscherharzes und danach eines Anionenaustauscherharzes demineralisiert wird, wobei bevorzugt mindestens 0,02 H⁺ Äquivalente pro Mol Pentosen verbleiben.

6. Verwendung der nach dem Verfahren des Anspruches 2 erhältlichen Pentosiden-Mischung, als oberflächenaktives Mittel insbesondere als Detergens und in der Kosmetologie.

7. Detergens-Zusammensetzung mit 0,1 bis 60 Gew.-% und bevorzugt 10 bis 30 Gew.-% einer Detergens-Base und 99,9 bis 40 Gew.-% und bevorzugt 90 bis 70 Gew.-% Adjuvantien, dadurch gekennzeichnet, daß die Detergens-Base 1 bis 100 Gew.-% und bevorzugt 50 bis 100 Gew.-% einer nach dem Verfahren des Anspruchs 2 erhältlichen Pentosidenmischung besteht.

8. Kosmetische Zusammensetzung mit 0,1 bis 50 Gew.-% und bevorzugt 5 bis 35 Gew.-% eines Tensids und 99,9 bis 50 Gew.-% und bevorzugt 95 bis 65 Gew.-% von Adjuvans oder Wirkstoff, dadurch gekennzeichnet, daß 0,5 bis 100 Gew.-% der Tenside aus der nach dem Verfahren des Anspruchs 2 erhältlichen Pentosidenmischung bestehen.
